# EUROPEAN PATENT SPECIFICATION

(11) **EP 2 450 047 B1**
(45) Date of publication and mention of the grant of the patent: **12.08.2015**
(21) Application number: 09846678.2
(22) Date of filing: 30.06.2009
(51) Int. Cl.: A61K 36/8888, A61K 36/78, A61K 36/752, A61K 36/736, A61K 36/634, A61K 36/54, A61K 36/539, A61K 36/535, A61K 36/484, A61K 36/28, A61K 36/076, A61P 11/06, A61P 11/08, A61K 33/06

(54) **PHARMACEUTICAL COMPOSITION CONTAINING EPHEDRAE FOR TREATING BRONCHITIS AND PREPARATION METHOD THEREFOR**
PHARMAZEUTISCHE ZUSAMMENSETZUNG MIT EPHEDRA ZUR BEHANDLUNG VON BRONCHITIS UND ZUBEREITUNGSVERFAHREN DAFÜR
COMPOSITION PHARMACEUTIQUE POUR TRAITER LA BRONCHITE QUI CONTIENT DE L'EPHEDRAE ET SON PROCÉDÉ DE PRÉPARATION

(43) Date of publication of application: 09.05.2012
(73) Proprietor: Hebei Yiling Medicine Research Institute Co., Ltd., Hebei 050035 (CN)
(72) Inventor: WU, Yiling, Shijiazhuang Hebei 050035 (CN); XU, Honghui, Shijiazhuang Hebei 050035 (CN); SONG, Honggang, Shijiazhuang Hebei 050035 (CN); LI, Wenlie, Shijiazhuang Hebei 050035 (CN); LI, Xiaoyan, Shijiazhuang Hebei 050035 (CN); WANG, Hongtao, Shijiazhuang Hebei 050035 (CN); ZHANG, Huixin, Shijiazhuang Hebei 050035 (CN); AN, Junyong, Shijiazhuang Hebei 050035 (CN); LI, Yunpeng, Shijiazhuang Hebei 050035 (CN); WANG, Chao, Shijiazhuang Hebei 050035 (CN)
(74) Representative: Kador & Partner
(86) International application number: PCT/CN2009/072531
(87) International publication number: WO 2011/000149

(56) References cited:
- CN-A- 1 775 266
- CN-A- 101 269 168
- ZHU YING; LIU XIAODONG: "Treatment of chronic bronchitis with modified ma xing shi gan tang and er chen tang.", JOURNAL OF TRADITIONAL CHINESE MEDICINE, vol. 24, no. 1, March 2004 (2004-03), pages 12-13, XP009167376,
- Anonymous: "Blue Poppy Press Recent Research Report 199: Cough", Blue Poppy Press , 1995, pages 1-2, XP002692750, Retrieved from the Internet: URL:http://bluepoppy.com/cfwebstore/index. cfm/feature/477/research-report-199-cough. cfm [retrieved on 2013-02-25]
- DI B ET AL: "Pharmacokinetic comparisons of Shuang-Huang-Lian with the different combinations of its constitutional herbs", JOURNAL OF ETHNOPHARMACOLOGY, vol. 107, no. 3, 11 October 2006 (2006-10-11), pages 401-405, XP027939592, ELSEVIER SCIENTIFIC PUBLISHERS LTD, IE ISSN: 0378-8741 [retrieved on 2006-10-11]
- DATABASE WPI Week 200482 Thomson Scientific, London, GB; AN 2004-822477 XP002692751, -& CN 1 528 391 A (ZHENGDA TIANQING PHARM CO LTD JIANGSU) 15 September 2004 (2004-09-15)
- DATABASE WPI Week 200239 Thomson Scientific, London, GB; AN 2002-353044 XP002692752, -& CN 1 336 214 A (CAO H) 20 February 2002 (2002-02-20)
- DATABASE WPI Week 200732 Thomson Scientific, London, GB; AN 2007-330115 XP002692753, -& CN 1 840 118 A (ZHANG Y) 4 October 2006 (2006-10-04)
- DATABASE WPI Week 200945 Thomson Scientific, London, GB; AN 2009-K84177 XP002692754, -& CN 101 455 822 A (LIU M) 17 June 2009 (2009-06-17)
- DATABASE WPI Week 200867 Thomson Scientific, London, GB; AN 2008-L32377 XP002692755, & CN 101 129 573 A (YIN K) 27 February 2008 (2008-02-27)
- DATABASE WPI Week 200525 Thomson Scientific, London, GB; AN 2005-234111 XP002692756, -& CN 1 557 432 A (SICHUAN RENDE PHARM CO LTD) 29 December 2004 (2004-12-29)
- DATABASE WPI Week 200627 Thomson Scientific, London, GB; AN 2006-254478 XP002692757, -& CN 1 679 864 A (ZHANG Y) 12 October 2005 (2005-10-12)
- DATABASE WPI Week 200632 Thomson Scientific, London, GB; AN 2006-300453 XP002692758, -& CN 1 706 467 A (GONG M) 14 December 2005 (2005-12-14)
- WU, ZHAOPING: 'Clinical Application of ''Maxing Shigan Decoction''' JOURNAL OF XINJIANG OF TRADITIONAL CHINESE MEDICINE 1994, pages 55 - 56, XP008148736
- BAO, QUANSHOU: 'A new study of Zhong Jin 's Maxing Shigan Decoction' JOURNAL OF PRACTICAL MEDICAL TECHNIQUES 1996, page 450, XP008148735
- HUANG, XIAOJU: 'Research progress of traditional Chinese medicine therapy for Child bronchitis' JOURNAL OF PEDIATRICS OF TRADITIONAL CHINESE MEDICINE 2007, pages 55 - 57, XP008148737

## Description

### Technical Field of the Invention

The invention belongs to the field of Traditional Chinese Medicine, in particular to a pharmaceutical composition for treatment of bronchitis and preparation methods thereof.

### Background of the Invention

Bronchitis is an inflammation in the mucous membrane of trachea and bronchi due to bacterial and viral infections, or stimulations by physical and chemical factors. Typically, it is mainly characterized by cough, expectoration, substernal malaise or pain, panting, and accompanying common symptom of cold. According to the disease duration, bronchitis can be classified into two categories: the acute tracheobronchitis, and the chronic bronchitis. The acute tracheobronchitis is a bronchitis which generally lasts for no more than one month and results in pathological changes only localized at the mucous membrane which can be completely restored in both of its structure and function after recovery. It may occur at any age and mostly in spring and winter. Bronchitis belongs to the categories of "cough", "phlegm-fluid retention", "asthma syndrome", etc, in the Traditional Chinese Medicine.

The acute tracheobronchitis is often caused by infection of the upper respiratory tract by viruses or bacteria, or by physical-chemical allergic stimuli. In patients of above 10 years old suffering from the acute tracheobronchitis, infections by influenza virus, respiratory syncytial virus and adenovirus are most common. This disease is also one of the most common non-hospital diseases and one of the top ten diseases in need of receiving medical treatment. There were 2.5 million Americans who received medical treatment due to the disease in 1998. Patients have to go to hospitals and receive treatment due to their normal work and lives being disturbed by frequent cough. Costs for treatment of acute tracheobronchitis are considerable. Averagely, each patient may be treated with two drugs and unable to work normally for 2 to 3 weeks.

The disease relates to various etiological factors. It may be caused by a direct infection of viruses or bacteria, or by an infection of viruses or bacteria spreading from acute upper respiratory tract infection; and may also be caused by an acute stimulation from such as over cold air, dust, an irritant gas or fumes to tracheal-bronchiaf mucus membrane; or by an allergen-induced allergic inflammatory response in trachea and bronchi. At present, the therapy from the Western Medicine focuses on symptomatic treatment and infection control. In the "Guidance For Rational Use Of Antibiotics In Acute Respiratory Tract Infections (For Trial)" issued in 2000 by the Subspecialty Group of Respiratory Diseases, the Society of Pediatrics, Chinese Medical Association, it is emphasized that the acute tracheobronchitis is the disease just after the upper respiratory tract infection (URI) for which antibiotics abuse usually occurs. In abroad, the acute tracheobronchitis is included into the cough illnesses and the major etiological factor for the disease is determined to be viruses or reactive airway diseases. Therefore, a patient with the course of the disease of less than 7 days has rarely indications applicable to antibiotic therapy. In addition, due to extensive application of antibacterials, drug-resistant strains emerge continually and a part of the pathogenic bacteria display multiple resistances to various antibacterials, which results in poor efficacy of antibiotics against the infection.

The treatment of acute tracheobronchitis has been a quit long history in Traditional Chinese Medicine. Systematical discussions on the issues of cough such as pathogenesis, symptoms and syndromes classification, pathological outcome and treatment can be found as early in Huangdi's Internal Classics. Continuous exploration has been lasting over generations of physicians on principles, methods, prescriptions and drugs for treatment of cough by Traditional Chinese Medicine. It is shown by the modern pharmacological investigation that treatment of cough by Traditional Chinese Medicine embodies various therapeutic approaches. The prescription of Traditional Chinese Medicine can not only exert effects of checking cough, eliminating phlegm, and relieving asthma, but also have effects of clearing heat, relieving pain, and resisting inflammation, as well as significantly relieve symptoms such as cough and expectoration. In addition, it has less adverse reactions in comparison with Western pharmaceuticals and is suitable for cases lacking of indications for antibiotic therapy, and particularly suitable for cases showing multiple resistances to antibiotics.

Therefore, there is still a need in developing novel Traditional Chinese Medicine prescriptions for treatment of acute tracheobronchitis.

### Summary of the Invention

To meet the need mentioned above, the present invention provides an EPHEDRAE HERBA-containing pharmaceutical composition for treatment of bronchitis and a preparation method thereof. The pharmaceutical composition of the present invention is made from the medicinal materials in the following parts by weight:

| | | | |
|---|---|---|---|
| EPHEDRAE HERBA 52-86 | GYPSUM FIBROSUM 194-324 | FORSYTHIAE FRUCTUS 194-324 | SCUTELLARIAE RADIX 78-130 |
| MORI CORTEX 194-324 | ARMENIACAE SEMEN AMARUM 78-130 | PEUCEDANI RADIX 78-130 | PINELLIAE RHIZOMA 78-130 |
| CITRI RETICULATAE PERICARPIUM 78-130 | FRITILLARIAE THUNBERGII BULBUS 78-130 | ARCTII FRUCTUS 78-130 | LONICERAE FLOS 78-130 |
| RHEI RADIX ET RHIZOMA 39-65 | PLATYCODONIS RADIX 46-76 | and GLYCYRRHIZAE RADIX ET RHIZOMA 39-65. | |

| | | | |
|---|---|---|---|
| (Note: the above Latin names of the medicinal materials are according to Chinese Pharmacopoeia, Jan., 2010.) | | | |

Preferably, the pharmaceutical composition of the present invention is made from the medicinal materials in the following parts by weight:

| | | | |
|---|---|---|---|
| EPHEDRAE HERBA 52 | GYPSUM FIBROSUM 324 | FORSYTHIAE FRUCTUS 194 | SCUTELLARIAE RADIX 78 |
| MORI CORTEX 194 | ARMENIACAE SEMEN AMARUM 130 | PEUCEDANI RADIX 78 | PINELLIAE RHIZOMA 130 |
| CITRI RETICULATAE PERICARPIUM 78 | FRITILLARIAE THUNBERGII BULBUS 78 | ARCTII FRUCTUS 130 | LONICERAE FLOS 130 |
| RHEI RADIX ET RHIZOMA 39 | PLATYCODONIS RADIX 76 | and GLYCYRPHIZAE RADIX ET RHIZOMA 65. | |

More preferably, the pharmaceutical composition of the present invention is made from the medicinal materials in the following parts by weight:

| | | | |
|---|---|---|---|
| EPHEDRAE HERBA 86 | GYPSUM FIBROSUM 194 | FORSYTHIAE FRUCTUS 324 | SCUTELLARIAE RADIX 130 |
| MORI CORTEX 324 | ARMENIACAE SEMEN AMARUM 78 | PEUCEDANI RADIX 130 | PINELLIAE RHIZOMA 78 |
| CITRI RETICULATAE PERICARPIUM 130 | FRITILLARIAE THUNBERGII BULBUS 130 | ARCTII FRUCTUS 78 | LONICERAE FLOS 130 |
| RHEI RADIX ET RHIZOMA 65 | PLATYCODONIS RADIX 46 | and GLYCYRRHIZAE RADIX ET RHIZOMA 39. | |

Alternatively, the pharmaceutical composition is made from the medicinal materials in the following parts by weight:

| | | | |
|---|---|---|---|
| EPHEDRAE HERBA 69 | GYPSUM FIBROSUM 259 | FORSYTHIAE FRUCTUS 259 | SCUTELLARIAE RADIX 104 |
| MORI CORTEX 259 | ARMENIACAE SEMEN AMARUM 104 | PEUCEDANI RADIX 104 | PINELLIAE RHIZOMA 104 |
| CITRI RETICULATAE PERICARPIUM 104 | FRITILLARIAE THUNBERGIIBULBUS 104 | ARCTII FRUCTUS 104 | LONICERAE FLOS 104 |
| RHEI RADIX ET RHIZOMA 52 | PLATYCODONIS RADIX 61 | and GLYCYRRHIZAE RADIX ET RHIZOMA 52. | |

Alternatively, the pharmaceutical composition is made from the medicinal materials in the following parts by weight:

| | | | |
|---|---|---|---|
| EPHEDRAE HERBA 55 | GYPSUM FIBROSUM 254 | FORSYTHIAE FRUCTUS 318 | SCUTELLARIAE RADIX 107 |
| MORI CORTEX 203 | ARMENIACAE SEMEN AMARUM 107 | PEUCEDANI RADIX 82 | PINELLIAE RHIZOMA 105 |
| CITRI RETICULATAE PERICARPIUM 84 | FRITILLARIAE THUNBERGII BULBUS 125 | ARCTII FRUCTUS 122 | LONICERAE FLOS 113 |
| RHEI RADIX ET RHIZOMA 42 | PLATYCODONIS RADIX 60 | and GLYCYRRHIZAE RADIX ET RHIZOMA 50. | |

Alternatively, the pharmaceutical composition is made from the medicinal materials in the following parts by weight:

| | | | |
|---|---|---|---|
| EPHEDRAE HERBA 84 | GYPSUM FIBROSUM 313 | FORSYTHIAE FRUCTUS 298 | SCUTELLARIAE RADIX 98 |
| MORI CORTEX 274 | ARMENIACAE SEMEN AMARUM 105 | PEUCEDANI RADIX 116 | PINELLlAE RHIZOMA 123 |
| CITRI RETICULATAE PERICARPIUM 95 | FRITILLARIAE THUNBERGII BULBUS 88 | ARCTII FRUCTUS 80 | LONICERAE FLOS 99 |
| RHEI RADIX ET RHIZOMA 63 | PLATYCODONIS RADIX 55 | and GLYCYRRHIZAE RADIX ET RHIZOMA 43. | |

For the medicinal materials used in the pharmaceutical composition of the present invention, PINELLIAE RHIZOMA is preferably PINELLIAE RHIZOMA PRAEPARATUM CUM ALUMINE and ARMENIACAE SEMEN AMARUM is preferably ARMENIACAE SEMEN AMARUM TOSTUM (the Latin name is according to Standard of Chinese Herbal Pieces of Shanxi Province, Feb., 2008).

EPHEDRAE HERBA and GYPSUM FIBROSUM in the pharmaceutical composition of the present invention are principal drugs. EPHEDRAE HERBA is warm in nature, pungent and bitter in taste, and enters the Lung and Urinary Bladder channels. It is described in Shen Nong's Herbal that "EPHEDRAE HERBA relieves exterior syndrome by diaphoresis, clears the pathogenic heat qi and checks cough with asthma." It is also described in Materia medica of Justice that "EPHEDRAE HERBA is light, clearing and floating in nature, specializes in removing pulmonary stagnation and faciliating the functional acitivity of qi and is the most essential drug for treating cold. Although EPHEDRAE HERBA is offered to release the exterior, it actually ventilates the lung. Although EPHEDRAE HERBA is offered to disperse cold, it actually expels the pathogenic factors. Therefore, wind-clod is always dispelled outwardly by EPHEDRAE HERBA, that is, warm-heat is always promoted by EPHEDRAE HERBA." EPHEDRAE HERBA in the prescription is used to ventilate the lung, thus facilitating the flow of stagnant qi in the lung, and to clear the heat in the lung in combination with GYPSUM FIBROSUM. GYPSUM FIBROSUM is pungent and sweet in taste, cold in nature, and enters the Lung and Stomach Channels. It is described in Ben Cao Jing Shu that "the pungent taste of GYPSUM FIBROSUM can be useful for releasing the muscle, and it's sweet taste for relieving the pathogenic heat. Therefore, GYPSUM FIBROSUM can be used to eliminate the serious heat due to its extreme cold property and its pungent and sweet tastes." GYPSUM FIBROSUM specializes in clearing heat and removing fire and can be useful for clearing heat in the lung in combination with EPHEDRAE HERBA. If heat is cleared, the production of phlegm will not be facilitated any more. Since the EPHEDRAE HERBA and the GYPSUM FIBROSUM is compatible with each other to ventilate the lung and clear away heat, they together act as the principal drugs.

FORSYTHIAE FRUCTUS, SCUTELLARIAE RADIX, MORI CORTEX, ARMENIACAE SEMEN AMARUM, and PEUCEDANI RADIX in the pharmaceutical composition of the present invention are minister drugs. FORSYTHIAE FRUCTUS is bitter in taste and cold in nature. It can externally relieve the muscle and the surface, and internally clear the stagnant heat, "has the capacity of floating and dissipating ...... can relieve the exterior syndromes through the muscle, clear heat and expel the wind. It is the essential drug for wind-heat pathogen" (Yi Xue Zhong Zhong Can Xi Lu). The heat-clearing, detoxication, lung-clearing and exterior thrusting functions of the FORSYTHIAE FRUCTUS is used in the prescription. SCUTELLARIAE RADIX is bitter in taste, cold in nature, enters the Lung Channel, acts on clearing away heat and drying dampness, punging fire for detoxication. SCUTELLARIAE RADIX specializes in clearing away pulmonary heat, for which Dan Xi had stated that "since SCUTELLARIAE RADIX serves for descending the phlegm, it can be used to dissipate fire" . The effect of clearing pulmonary heat will be enhanced with the aid of GYPSUM FIBROSUM. MORI CORTEX is sweet in taste, cold in nature, and enters the Lung and Spleen Channels. MORI CORTEX has functions of clearing the lung and resolving phlegm, as wellas descending *qi* and relieving asthma, which is described in Bie Lu: "removing the dampness in the lung, spitting blood, pyretic thirst, edema and abdominal fullness, belly swelling, regulating *waterway* and expelling the taenia". SCUTELLARIAE RADIX and MORI CORTEX enhance the effect of GYPSUM FIBROSUM in clearing phlegme-heat in the lung. ARMENIACAE SEMEN AMARUM is mild warm in nature, slightly bitter in taste, enters the Lung and large Intestinal Channels, and serves for checking cough and relieving asthma. ARMENIACAE SEMEN AMARUM is effective in checking cough and relieving asthma by purge and directing qi downward, "specializing in directing *qi* downward, wherein the phlegm is eliminated and cough is checked upon *qi* is directed downward"(Ben Cao Bian Du). PEUCEDANI RADIX is bitter and pungent in taste, mild codl in nature, enters the Lung MOeridian, and serves for directing *qi* downward, dispelling phlegm, and clearing wind-heat. In Compendium of Materia Medica, it has been described to clear pulmonary heat, resolving phlegm-heat, dispersing pathogenic wind", and futher described that "it specializes in directing qi downward, and thus can be used to treat diseases such as asthmaitc cough induced by phlegm-heat and diaphragm stuffiness with hiccup. When *qi* is directed downward, the fire is removed and the phlegm is resolving". The combination of ARMENIACAE SEMEN AMARUM and PEUCEDANI RADIX (specializing in lowering) with EPHEDRAE HERBA (specializing in lifting) enhances the dispersing and descending functions of the lung. These five herbs function together as minister drugs to facilitate the pulmonary-heat-clearing effect of the principal drugs by relieving both the interior and exterior, and descending *qi* and dispelling phlegm.

PINELLIAE RHIZOMA, CITRI RETICULATAE PERICARPIUM, FRITILLARIAE THUNBERGII BULBUS, ARCTII FRUCTUS, LONICERAE FLOS, and RHEI RADIX ET RHIZOMA in the pharmaceutical composition of the present invention are assistant drugs. PINELLIAE RHIZOMA is pungent in taste, warm in nature, serves for eliminating dampness by dryness, resolving phlegm and calming the adverse-rising energy. In Yao Xing Lun, PINELLIAE RHIZOMA is described to specializing in "dispersing phlegm, discending lung qi, increasing appetite and invigorating spleen, and dissipating excessive phlegm in the chest", that is, PINELLIAE RHIZOMA is the essential drug for resolving phlegm and calming the adverse-rising energy and exerts its efficacy downward to direct the damp phlegm in the lung and stomach moving downward. CITRI RETICULATAE PERICARPIUM is pungent and bitter in taste, warm in nature, and serves for regulating *qi* and resolving phlegm. "Tangerine pericarp (CITRI RETICULATAE PERICARPIUM) mainly acts on the agglomerated heat and the adverse flow of *qi* in the chest, ...... the pungent taste is capable of dispersing, the bitter taste purging, and the warm property moving, thus descending the adversely rising qi, preventing vomit and checking cough, eventually disspating the agglomerated heat in the chest (Ben Cao Jing Shu)". CITRI RETICULATAE PERICARPIUM, is the common herb for treating stagnation of dampness-phlegm, impairment of dispersing and descending function of the lung, cough, excessive phlegm, and *qi* regurgitating. The combination of PINELLIAE RHIZOMA and CITRI RETICULATAE PERICARPIUM assist the principal and minister drugs to reinforce the phlegm-resolving effect. FRITILLARIAE THUNBERGII BULBUS is bitter in taste, cold in nature, enters Lung and Heart channels, and often used to treat cough of stagnated phlegme-heat type due to its more bitterness and coldness, potent purging power, stronger effects of clearing away fire and dispersing masses. It is used in the prescription to facilitate ARMENIACAE SEMEN AMARUM and PEUCEDANI RADIX to resolve phlegm and check cough. ARCTII FRUCTUS is pungent in taste, bitter in taste, mild cool in nature, and specialize in dispersing wind-heat pathogen, and it is described in Ben Cao Jing Shu that: "aversion to the excess, a primary herb for dispersing wind, removing heat and detoxicating". LONICERAE FLOS is cold in nature and able to clear heat and detoxicate. It is described in Chong Qing Tang Sui Bi that "LONICERAE FLOS clears away the wind-fire and excessive heat in the collaterals and eliminate evils such as pest, filthy, and turbid." The herb enters the Lung Channel. It is able to clear heat and detoxicate, and also to clear the lung and promoting perspiration. Being pungent and cold in taste and no harmful to *Yin,* LONICERAE FLOS assists FORSYTHIAE FRUCTUS to attain perspiration, heat-clearing and detoxication with pungent and cool properties. The RHEI RADIX ET RHIZOMA is bitter in taste, cold in nature, serves for unblocking the bowels and punging the heat. Due to the lung and the large intestine being interior-exteriorly related, lung *qi* decends spontaneously upon bowel qi is purged. Thus, the combination of RHEI RADIX ET RHIZOMA and EPHEDRAE HERBA results in reliveing both of the exterior and interior.

PLATYCODONIS RADIX and GLYCYRRHIZAE RADIX ET RHIZOMA in the pharmaceutical composition of the present invention are guide drugs. PLATYCODONIS RADIX serves for clearing lung *qi* and dispelling phlegm and thus assists EPHEDRAE HERBA in clearing lung qi. Moreover, "PLATYCODONIS RADIX is a herb for clearing and lifting lung qi and able to act as a carrier for various herbs" (Ben Cao Qiu Zhen). The herb transports the various herbal ingredients to reach the pathological site, thus serving as an assistant-guide drug. GLYCYRRHIZAE RADIX ET RHIZOMA is used in its crude form for two reasons. The first reason is that GLYCYRRHIZAE RADIX ET RHIZOMA as an assistant drug is sweet and neutral in nature, can enter the Lung Channel and has an effect of moisturizing the lung, checking cough and relieving asthma. The second reason is that as a guide drug, "GLYCYRRHIZAE RADIX ET RHIZOMA plays a role of a senior coordinator for a variety of herbs" and can attemper the drugs of the prescription, thus also serves as an assistant and guide drug.

In conclusion, the combined use of the medicinal substances constituting the pharmaceutical composition of the present invention results in clearing phlegm-heat in lung, dispersing stagnated lung qi, and normalizing dispersing and descending function of the lung, thereby removing phlegm and checking cough.

In the light of the present invention and in combination with the common sense in the field, those skilled in the art may replace any of the individual components in the pharmaceutical composition of the present invention with other Chinese medicinal material having same or similar efficacies as the replaced component in the present pharmaceutical composition.

The pharmaceutical composition of the present invention can be prepared with the conventional preparation method in the art. For example, the various medicinal substances can be processed in accordance with "Standard for Processing Traditional Chinese Medicinal Substances" or "Traditional Chinese Medicine Dictionary". The pharmaceutical composition of the present invention can be prepared preferably with the following method:
(1) weighing FRITILLARIAE THUNBERGII BULBUS according to the part set forth in the prescription and pulverizing the same into fine powder for later use;
(2) weighing EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA according to the parts set forth in the prescription and extracting the same two times with 40-70% ethanol solution under reflux for 1-4 hours each time, in which the amount of the ethanol solution may be 8-10 times by v/w (ml/g) of the weight of the medicinal materials for the first time and 6-9 times by v/w (ml/g) in the second time; pooling the extracts and then filtrating the mixed solution; recovering ethanol from the filtrate under reduced pressure and concentrating at the same time to obtain a clear paste with the relative density of 1.14-1.16 thermally measured at 60 °C for later use;
(3) weighing GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA according to the parts set forth in the prescription and decocting the same two times with water for 1-4 hours each time, in which the amount of water may be 9-11 times by v/w (ml/g) of the weight of the medicinal materials for the first time and 7-9 times by v/w (ml/g) for the second time; then, pooling the decoctions and filtrating, concentrating the filtrate under reduced pressure to obtain a clear paste with the relative density of 1.14-1.16 thermally measured at 60°C, and combining the clear past with the clear paste obtained in step (2) for later use;

The fine powder obtained in step (1) and the combined clear paste obtained in step (3) constitute the active components of the pharmaceutical composition.

Dosage forms of the pharmaceutical composition according to the invention may be hard capsule, tablet, powder, oral liquid, soft capsule, pill, tincture, syrup, suppository, gel, spray, or injection.

The dosage forms of the present medication can be prepared into pharmaceutically acceptable conventional dosage forms with the preparation method common in the art, for example, such as the preparation processes recorded in Pharmaceutics of Chinese Medicine, Fan Biting (the Editor), 1997, Shanghai Scientific & Technical Publishers.

In formulating the various pharmaceuticals of the present invention, a person skilled in the art may add any of pharmaceutically acceptable auxiliary materials depending on the dosage forms of the different medications of the present invention and in combination with the common sense in the art, for example, the auxiliary materials include, but are not limited to a filler, a disintegrant, a lubricant, a suspending agent, a binder, a sweetener, a flavoring agent, a preservative, a matrix, or any of mixtures thereof,. The filler includes, but is not limited to starch, pregelatinized starch, lactose, mannitol, chitin, microcrystalline cellulose, sucrose, or any of mixtures thereof. The disintegrants includes, but is not limited to starch, pregelatinized starch, microcrystalline cellulose, sodium carboxymethyl starch, cross-linked polyvinylpyrrolidone, low substituted hydroxypropyl cellulose, crossl-inked sodium carboxymethyl cellulose, or any of mixtures thereof. The lubricant includes, but is not limited to magnesium stearate, sodium lauryl sulfate, talc, silica, or any of mixtures thereof. The suspending agent includes, but is not limited to polyvinylpyrrolidone, microcrystalline cellulose, sucrose, agar, hydroxypropyl methyl cellulose, or any of mixtures thereof. The binder includes, but is not limited to starch slurry, polyvinylpyrrolidone, hydroxypropyl methyl cellulose, or any of mixtures thereof. The sweetener includes, but is not limited to sodium saccharin, aspartame, sucrose, sodium cyclamate, glycyrrhetinic acid, or any of mixtures thereof. The flavoring agent includes, but is not limited to sweeteners, various essences, or any of mixtures thereof. The preservative includes, but is not limited to: parabens, benzoic acid, sodium benzoate, sorbic acid and salts thereof, benzalkonium bromide, chlorhexidine acetate, eucalyptus oil, or any of mixtures thereof. The matrix includes, but is not limited to PEG6000, PEG4000, insect wax, or any of mixtures thereof. In order to prepare the above-described dosage forms according to pharmaceutics of Traditional Chinese Medicine, other auxiliary materials which are pharmaceutically acceptable (e.g. auxiliary materials for various dosage forms recorded in Pharmaceutics of Chinese Medicine, Fan Biting (the Editor), 1997, Shanghai Scientific & Technical Publishers) may further be added when preparing those dosage forms.

As a preferred embodiment, the pharmaceutical composition of the present invention is prepared with the following method:
(1) weighing FRITILLARIAE THUNBERGII BULBUS according to the part set forth in the prescription and pulverizing the same into a fine powder for later use;
(2) weighing EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA according to the parts set forth in the prescription and extracting the same two times with 40-70% ethanol solution under reflux for 1-4 hours each time, in which the amount of the ethanol solution may be 8-10 times by v/w (ml/g) of the weight of the medicinal materials for the first time and 6-9 times by v/w (ml/g) for the second time; pooling the extracts and filtrating the mixed solution; recovering ethanol from the filtrate under reduced pressure and concentrating at the same time to obtain a clear paste with the relative density of 1.14-1.16 thermally measured at 60°C, for later use;
(3) weighing GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA according to the parts set forth in the prescription and decocting the same two times with water for 1-4 hours each time, in which the amount of the added water may be 9-11 times by v/w (ml/g) of the weight of the medicinal materials for the first time and 7-9 times by v/w (ml/g) for the second time; then, pooling the decoctions and filtrating the mixed solution, concentrating the filtrate under reduced pressure to obtain a clear paste with the relative density of 1.14-1.16 thermally measured at 60 °C, and combining the clear paste with the clear paste obtained in step (2) for later use;
(4) spray drying the combined clear pastes obtained in step (3) and collecting the atomized powder for later use;
(5) providing materials (parts by weight) for tebleting as follows:

| | |
|---|---|
| atomized powder obtained in step (4) 200-355; | fine powder obtained in step (1) 54-145; |
| carboxymethylstarch sodium 10-15.5; | microcrystalline cellulose 7-12; |
| magnesium stearate 1.5-3.5; | starch proper amount; |

(6) tableting according to conventional formulation process.

As a further preferred embodiment, the pharmaceutical composition of the present invention is prepared with the following method:
(1) weighing FRITILLARIAE THUNBERGII BULBUS according to the part set forth in the prescription and pulverizing the same into a fine powder for later use;
(2) weighing EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA according to the parts set forth in the prescription and extracting the same two times with 50% ethanol solution under reflux for 3 hours each time, in which the amount of the ethanol solution is 10 times by v/w (ml/g) of the weight of the medicinal materials for the first time and 6 times by v/w (ml/g) for the second time; pooling the extracts and filtrating the mixed solution; recovering ethanol from the filtrate under reduced pressure and concentrating at the same time to obtain a clear paste with the relative density of 1.15 thermally measured at 60°C, for later use;
(3) weighing GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA according to the parts set forth in the prescription and decocting the same two times with water for 2 hours each time, in which the amount of the added water is 10 times by v/w (ml/g) of the weight of medicinal materials for the first time and 7 times by v/w (ml/g) for the second time; then, pooling the decoctions and filtrating the mixed solution, concentrating the filtrate under reduced pressure to obtain a clear paste with the relative density of 1.15 thermally measured at 60 °C, and combining the clear past with the clear paste obtained in step (2) for later use;
(4) spray drying the combined clear pastes obtained in step (3) and collecting the atomized powder for later use;
(5) providing materials (parts by weight) for tebleting as follows:

| | |
|---|---|
| atomized powder obtained in step (4) 282.6; | fine powder obtained in step (1) 101; |
| carboxymethylstarch sodium 12.5; | microcrystalline cellulose 9.0; |
| magnesium stearate 2.25; | starch proper amount; |

(6) tableting according to a conventional formulation process.

The pharmaceutical composition of the present invention was achieved on the basis of the collateral disease theory from Traditional Chinese Medicine in combination with the modern medical investigation on the etiology and pathogenesis of the bronchitis, as well as the results from modern pharmacological studies on the analysis of constitutive herbs and the clinical practices. The pharmaceutical composition has effects of clearing the pulmonary heat, resolving phlegm and checking cough, and exerts good efficacies in treating bronchitis, particularly excellent efficacy in treating acute tracheobronchitis.

It has been shown experimentally that the present medication has effects of checking cough, dispelling phlegm, relieving asthma, relieving heat and resisting inflammation and shows particularly excellent efficacy in treating acute tracheobronchitis. Moreover, the present medication has been shown experimentally to be free of side effects and safe.

### Detailed Description of the Invention

The present invention will be further illustrated by the following Examples and Test Examples which are not intended to limit the scope of the present invention in any way.

### Example 1 Preparation method for tablets of the present medication (tentatively named as: Lian Hua Ji Zhi tablet)

### Prescription:

| | | | |
|---|---|---|---|
| EPHEDRAE HERBA 52 g | GYPSUM FIBROSUM 324 g | FORSYTHIAE FRUCTUS 194 g | SCUTELLARIAE RADIX 78 g |
| MORI CORTEX 194 g | ARMENIACAE SEMEN AMARUM 130 g | PEUCEDANI RADIX 78 g | PINELLIAE RHIZOMA 130 g |
| CITRI RETICULATAE PERICARPIUM 78 g | FRITILLARIAE THUNBERGII BULBUS 78 g | ARCTII FRUCTUS 130 g | LONICERAE FLOS 130 g |
| RHEI RADIX ET RHIZOMA 39 g | PLATYCODONIS RADIX 76 g | GLYCYRRHIZAE RADIX ET RHIZOMA 65 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times with 60% ethanol solution under reflux for 2 hours each time, in which the amount of the ethanol solution was 9 times by v/w (ml/g) of the weight of the medicinal materials for the first time and 8 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 2 hours, in which the amount of the water was 10 times by v/w (ml/g) of the weight of the medicinal materials for the first time and 7 times by v/w (ml/g) for the second time; then, the decoctions were pooled, filtrated, and the filtrate was concentrated under reduced pressure to a clear paste with the relative density of 1.15 thermally measured at 60 °C, which was combined with the clear paste obtained in step (2) for later use;
(4) the combined clear paste obtained in step (3) was spray dried and the atomized powder was collected for later use;
(5) the raw materials used for tablet consists of the following medicinal substances (parts by weight):
   the atomized powder obtained in step (4) 285.5;
   the raw medicine powder obtained in step (1) 105.4;
   carboxymethylstarch sodium 12.5;
   microcrystalline cellulose 8.5;
   magnesium stearate 2.45;
   starch proper amount;
(6) tablets were prepared according to conventional formulation process.

### Example 2 Preparation method for capsules of the present medication

### Prescription:

| | | | |
|---|---|---|---|
| EPHEDRAE HERBA 86 g | GYPSUM FIBROSUM 194 g | FORSYTHIAE FRUCTUS 234 g | SCUTELLARIAE RADIX 130 g |
| MORI CORTEX 324g | ARMENIACAE SEMEN AMARUM 78 g | PEUCEDANI RADIX 130 g | PINELLIAE RHIZOMA 78 g |
| CITRI RETICULATAE PERICARPIUM 130 g | FRITILLARIAE THUNBERGII BULBUS 78 g | ARCTII FRUCTUS 78 g | LONICERAE FLOS 78 g |
| RHEI RADIX ET RHIZOMA 65 g | PLATYCODONIS RADIX 46 g | GLYCYRRHIZAE RADIX ET RHIZOMA 39 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times with 50% ethanol solution under reflux, each time for 2 hours, in which the amount of the ethanol solution was 8 times by v/w (ml/g) of the weight of the medicinal materials for the first time and 6 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 2 hours, in which the amount of the water is 9 times by v/w (ml/g) of the weight of the medicinal materials for the first time and 7 times by v/w (ml/g) for the second time; then, the decoctions were pooled, filtrated, the filtrate was concentrated under reduced pressure to a clear paste with the relative density of 1.14 thermally measured at 60 °C, which was combined with the clear paste obtained in step (2) for later use;
(4) capsules were prepared according to conventional formulation process.

### Example 3 Preparation method for powder of the present medication

### Prescription:

| | | | |
|---|---|---|---|
| EPHEDRAE HERBA 69 g | GYPSUM FIBROSUM 259 g | FORSYTHIAE FRUCTUS 259 g | SCUTELLARIAE RADIX 104 g |
| MORI CORTEX 259 g | ARMENIACAE SEMEN AMARUM 104 g | PEUCEDANI RADIX 104 g | PINELLIAE RHIZOMA 104 g |
| CITRI RETICULATAE PERICARPIUM 104 g | FRITILLARIAE THUNBERGII BULBUS 104 g | ARCTII FRUCTUS 104 g | LONICERAE FLOS 104 g |
| RHEI RADIX ET RHIZOMA 52 g | PLATYCODONIS RADIX 61 g | GLYCYRRHIZAE RADIX ET RHIZOMA 52 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times with 50% ethanol solution under reflux, each time for 2 hours, in which the amount of the ethanol solution was 8 times by v/w (ml/g) of the weight of the medicinal materials for the first time and 7 times by v/w (ml/g) for the second time; the extracts were pooled and filtrated; the filtrate was subjected to recovery of ethanol under reduced pressure and concentrated to a clear paste with the relative density of 1.14 - 1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 2 hours, in which the amount of the added water was 9 times by v/w (ml/g) of the weight of the medicinal materials for the first time and 8 times by v/w (ml/g) for the second time; then, the decoctions were pooled, filtrated, the filtrate was concentrated under reduced pressure to a clear paste with the relative density of 1.14 thermally measured at 60°C, and combined with the clear paste obtained in step (2) for later use;
(4) powder was prepared according to conventional formulation process.

### Example 4 Preparation method for oral liquor of the present medication

### Prescription:

| | | | |
|---|---|---|---|
| EPHEDRAE HERBA 55 g | GYPSUM FIBROSUM 254 g | FORSYTHIAE FRUCTUS 318 g | SCUTELLARIAE RADIX 107 g |
| MORI CORTEX 203 g | ARMENIACAE SEMEN AMARUM 107 g | PEUCEDANI RADIX 82 g | PINELLIAE RHIZOMA 105 g |
| CITRI RETICULATE PERICARPIUM 84 g | FRITILLARIAE THUNBERGII BULBUS 125 g | ARCTII FRUCTUS 122 g | LONICERAE FLOS 113 g |
| RHEI RADIX ET RHIZOMA 42 g | PLATYCODONIS RADIX 60 g | GLYCYRRHIZAE RADIX ET RHIZOMA 50 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 40% ethanol solution, each time for 1 hour, in which the amount of the ethanol solution was 8 times by v/w (ml/g) of the weight of the medicinal materials for the first time and 6 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60°C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 1 hour, in which the amount of the added water was 9 times by v/w (ml/g) of the weight of the medicinal materials for the first time and 7 times by v/w (ml/g) for the second time; then, the decoctions were pooled, filtrated, the filtrate was concentrated under reduced pressure to a clear paste with the relative density of 1.14-1.16 thermally measured at 60°C, and combined with the clear paste obtained in step (2) for later use;
   the fine powder obtained in step (1) and the combined clear pastes obtained in step (3) were prepared to oral liquor according to conventional formulation process.

### Example 5 Preparation method for soft capsules of the present medication

### Prescription:

| | | | |
|---|---|---|---|
| EPHEDRAE HERBA 84 g | GYPSUM FIBROSUM 313 g | FORSYTHIAE FRUCTUS 298 g | SCUTELLARIAE RADIX 98 g |
| MORI CORTEX 274 g | ARMENIACAE SEMEN AMARUM 105 g | PEUCEDANI RADIX 116 g | PINELLIAE RHIZOMA 123 g |
| CITRI RETICULATAE PERICARPIUM 95 g | FRITILLARIAE THUNBERGII BULBUS 88 g | ARCTI] FRUCTUS 80 g | LONICERAE FLOS 99 g |
| RHEI RADIX ET RHIZOMA 63 g | PLATYCODONIS RADIX 55 g | GLYCYRRHIZAE RADIX ET RHIZOMA 43 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 70% ethanol solution, each time for 4 hours, in which the amount of the ethanol solution was 10 times by v/w (ml/g) of the weight of the medicinal materials for the first time and 9 times by v/w (ml/g) for the second time; the extracts were pooled and filtrated; the filtrate was subjected to recovery of ethanol under reduced pressure and the filtrate was concentrated to a clear paste with the relative density of 1.14-1.16 thermally measured at 60°C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 4 hours, in which the amount of the added water was 11 times by v/w (ml/g) of the weight of the medicinal materials for the first time and 9 times by v/w (ml/g) for the second time; then, the decoctions were pooled, filtrated, the filtrate was concentrated under reduced pressure to a clear paste with the relative density of 1.14-1.16 thermally measured at 60 °C, and combined with the clear paste obtained in step (2) for later use;
   the fine powder obtained in step (1) and the combined clear pastes obtained in step (3) were prepared to soft capsules according to conventional formulation process.

### Example 6 Preparation method for pills of the present medication

### Prescription:

| | | | |
|---|---|---|---|
| EPHEDRAE HERBA 5 g | GYPSUM FIBROSUM 202 g | FORSYTHIAE FRUCTUS 213 g | SCUTELLARIAE RADIX 84 g |
| MORI CORTEX 235 g | ARMENIACAE SEMEN AMARUM 95 g | PEUCEDANI RADIX 106 g | PINELLIAE RHIZOMA 117 g |
| CITRI RETICULATAE PERICARPIUM 128 g | FRITILLARIAE THUNBERGII BULBUS 116 g | ARCTII FRUCTUS 105 g | LONICERAE FLOS 98 g |
| RHEI RADIX ET RHIZOMA 41 g | PLATYCODONIS RADIX 54 g | GLYCYRRHIZAE RADIX ET RHIZOMA 62 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 60% ethanol solution, each time for 3 hours, in which the amount of the ethanol solution was 9 times by v/w (ml/g) of the weight of the medicinal materials for the first time and 8 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60°C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 2.5 hours, in which the amount of the added water was 10 times by v/w (ml/g) of the weight of the medicinal materials for the first time and 8 times by v/w (ml/g) for the second time; then, the decoctions were pooled, filtrated, the filtrate was concentrated under reduced pressure to a clear paste with the relative density of 1.14-1.16 thermally measured at 60°C, and combined with the clear paste obtained in step (2) for later use;
   the fine powder obtained in step (1) and the combined clear pastes obtained in step (3) were prepared to pills according to conventional formulation process.

### Example 7 Preparation method for tincture of the present medication

### Prescription:

| | | | |
|---|---|---|---|
| EPHEDRAE HERBA 53 g | GYPSUM FIBROSUM 248 g | FORSYTHIAE FRUCTUS 253 g | SCUTELLARIAE RADIX 101 g |
| MORI CORTEX 264 g | ARMENIACAE SEMEN AMARUM 102 g | PEUCEDANI RADIX 103 g | PINELLIAE RHIZOMA 104 g |
| CITRI RETICULATAE PERICARPIUM 105 g | FRITILLARIAE THUNBERGII BULBUS 106 g | ARCTII FRUCTUS 107 g | LONICERAE FLOS 108 g |
| RHEI RADIX ET RHIZOMA 65 g | PLATYCODONIS RADIX 48 g | GLYCYRRHIZAE RADIX ET RHIZOMA 65 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 50% ethanol solution, each time for 3.5 hours, in which the amount of the ethanol solution was 10 times by v/w (ml/g) of the weight of the medicinal materials for the first time and 9 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 3 hours, in which the amount of the added water was 10 times by v/w (ml/g) of the weight of the medicinal materials for the first time and 7 times by v/w (ml/g) for the second time; then, the decoctions were pooled, filtrated, the filtrate was concentrated under reduced pressure to a clear paste with the relative density of 1.14-1.16 thermally measured at 60°C, and combined with the clear paste obtained in step (2) for later use;
   the fine powder obtained in step (1) and the combined clear pastes obtained in step (3) were prepared to tincture according to conventional formulation process.

### Example 8 Preparation method for syrup of the present medication

### Prescription:

| | | | |
|---|---|---|---|
| EPHEDRAE HERBA 86 g | GYPSUM FIBROSUM 324 g | FORSYTHIAE FRUCTUS 205 g | SCUTELLARIAE RADIX 84 g |
| MORI CORTEX 226 g | ARMENIACAE SEMEN AMARUM 85 g | PEUCEDANI RADIX 84 g | PINELLIAE RHIZOMA 85 g |
| CITRI RETICULATAE PERICARPIUM 93 g | FRITILLARIAE THUNBERGII BULBUS 95 g | ARCTII FRUCTUS 93 g | LONICERAE FLOS 95 g |
| RHEI RADIX ET RHIZOMA 42 g | PLATYCODONIS RADIX 49 g | GLYCYRRHIZAE RADIX ET RHIZOMA 39 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 70% ethanol solution, each time for 1 hour, in which the amount of the ethanol solution was 8 times by v/w (ml/g) of the weight of the medicinal materials for the first time and 6 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 1 hour, in which the amount of the added water was 11 times by v/w (ml/g) of the weight of the medicinal materials for the first time and 9 times by v/w (ml/g) for the second time; then, the decoctions were pooled, filtrated, the filtrate was concentrated under reduced pressure to a clear paste with the relative density of 1.14-1.16 thermally measured at 60 °C, and combined with the clear paste obtained in step (2) for later use;
   the fine powder obtained in step (1) and the combined clear pastes obtained in step (3) were prepared to syrup by conventional formulation process.

### Example 9 Preparation method for suppository of the present medication

### Prescription:

| | | | |
|---|---|---|---|
| EPHEDRAE HERBA 52 g | GYPSUM FIBROSUM 310 g | FORSYTHIAE FRUCTUS 312g | SCUTELLARIAE RADIX 121 g |
| MORI CORTEX 318 g | ARMENIACAE SEMEN AMARUM 122 g | PEUCEDANI RADIX 124 g | PINELLIAE RHIZOMA 126 g |
| CITRI RETICULATE PERICARPIUM 128 g | FRITILLARIAE THUNBERGII BULBUS 118 g | ARCTII FRUCTUS 116 g | LONICERAE FLOS 119 g |
| RHEI RADIX ET RHIZOMA 62 g | PLATYCODONIS RADIX 71 g | GLYCYRRHIZAE RADIX ET RHIZOMA 39 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 40% ethanol solution, each time for 4 hours, in which the amount of the ethanol solution was 10 times by v/w (ml/g) of the weight of the medicinal materials for the first time and 9 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 1 hour, in which the amount of the added water was 11 times by v/w (ml/g) of the weight of the medicinal materials for the first time and 9 times by v/w (ml/g) for the second time; then, the decoctions were pooled, filtrated, the filtrate was concentrated under reduced pressure to a clear paste with the relative density of 1.14-1.16 thermally measured at 60 °C, and combined with the clear paste obtained in step (2) for later use;
   the fine powder obtained in step (1) and the combined clear pastes obtained in step (3) were prepared to suppository by conventional formulation process.

### Example 10 Preparation method for gel of the present medication

### Prescription:

| | | | |
|---|---|---|---|
| EPHEDRAE HERBA 82 g | GYPSUM FIBROSUM 205 g | FORSYTHIAE FRUCTUS, 305 g | SCUTELLARIAE RADIX 82 g |
| MORI CORTEX 305 g | ARMENIACAE SEMEN AMARUM 82 g | PEUCEDANI RADIX 126 g | PINELLIAE RHIZOMA 88 g |
| CITRI RETICULATAE PERICARPIUM 124 g | FRITILLARIAE THUNBERGII BULBUS 85 g | ARCTII FRUCTUS 124 g | LONICERAE FLOS 85 g |
| RHEI RADIX ET RHIZOMA 64 g | PLATYCODONIS RADIX 48 g | GLYCYRRHIZAE RADIX ET RHIZOMA 64 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 55% ethanol solution, each time for 2.5 hours, in which the amount of the ethanol solution was 7 times by v/w (ml/g) of the weight of the medicinal materials for the first time and 9 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60°C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 3 hours, in which the amount of the added water was 9 times by v/w (ml/g) of the weight of the medicinal materials for the first time and 7 times by v/w (ml/g) for the second time; then, the decoctions were pooled, filtrated, the filtrate was concentrated under reduced pressure to a clear paste with the relative density of 1.14-1.16 thermally measured at 60°C, and combined with the clear paste obtained in step (2) for later use;
   the fine powder obtained in step (1) and the combined clear pastes obtained in step (3) were prepared to gel by conventional formulation process.

### Example 11 Preparation method for spray of the present medication

### Prescription:

| | | | |
|---|---|---|---|
| EPHEDRAE HERBA 65 g | GYPSUM FIBROSUM 234 g | FORSYTHIAE FRUCTUS 245 g | SCUTELLARIAE RADIX 84 g |
| MORI CORTEX 321 g | ARMENIACAE SEMEN AMARUM 118 g | PEUCEDANI RADIX 85 g | PINELLIAE RHIZOMA 125 g |
| CITRI RETICULATAE PERICARPIUM 90 g | FRITILLARIAE THUNBERGII BULBUS 94 g | ARCTII FRUCTUS 85 g | LONICERAE FLOS 97 g |
| RHEI RADIX ET RHIZOMA 55 g | PLATYCODONIS RADIX 55 g | GLYCYRRHIZAE RADIX ET RHIZOMA 55 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 60% ethanol solution, each time for 2.5 hours, in which the amount of the ethanol solution was 9 times by v/w (ml/g) of the weight of the medicinal materials for the first time and 6 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60°C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 3 hours, in which the amount of the added water was 9 times by v/w (ml/g) of the weight of the medicinal materials for the first time and 7 times by v/w (ml/g) for the second time; then, the decoctions were pooled, filtrated, concentrated under reduced pressure to a clear paste with the relative density of 1.14-1.16 thermally measured at 60°C, and combined with the clear paste obtained in step (2) for later use;
   the fine powder obtained in step (1) and the combined clear pastes obtained in step (3) were prepared to spray by conventional formulation process.

### Example 12 Preparation method for injection of the present medication Prescription:

| | | | |
|---|---|---|---|
| EPHEDRAE HERBA 73 g | GYPSUM FIBROSUM 205 g | FORSYTHIAE FRUCTUS 285 g | SCUTELLARIAE RADIX114 g |
| MORI CORTEX 275 g | ARMENIACAE SEMEN AMARUM 82 g | PEUCEDANI RADIX 123 g | PINELLIAE RHIZOMA 88 g |
| CITRI RETICULATAE PERICARPIUM 126 g | FRITILLARIAE THUNBERGII BULBUS 97 g | ARCTII FRUCTUS 119 g | LONICERAE FLOS 101 g |
| RHEI RADIX ET RHIZOMA 42 g | PLATYCODONIS RADIX 51 g | GLYCYRRHIZAE RADIX ET RHIZOMA 45 g | |

### Preparation method:

(1) FRITILLARIAE THUNBERGII BULBUS was weighed according to the weight of the prescription and pulverized into a fine powder for later use;
(2) EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA were weighed according to the weight of the prescription and extracted two times under reflux with 60% ethanol solution, each time for 3 hours, in which the amount of the ethanol solution was 9 times by v/w (ml/g) of the weight of the medicinal materials for the first time and 6 times by v/w (ml/g) for the second time; the extracts were pooled and filtrated; the filtrate was subjected to recovery of ethanol under reduced pressure and concentrated to a clear paste with the relative density of 1.14-1.16 thermally measured at 60 °C, for later use;
(3) GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA were weighed according to the weight of the prescription and decocted two times with water, each time for 1 hour, in which the amount of the added water was 9 times by v/w (ml/g) of the weight of the medicinal materials for the first time and 7 times by v/w (ml/g) for the second time; then, the decoctions were pooled, filtrated, concentrated under reduced pressure to a clear paste with the relative density of 1.14-1.16 thermally measured at 60 °C, and combined with the clear paste obtained in step (2) for later use;
   the fine powder obtained in step (1) and the combined clear pastes obtained in step (3) were prepared to injection by conventional formulation process.

All of the pharmaceutical compositions of the present invention administrated in the following Test Examples of pharmacological and toxicologic experiments (hereinafter referred to as "the present medication") were prepared with the procedure set forth in Example 1.

### Test Example 1 Experiments on cough-checking effect of the present medication

### 1. The cough-checking effect of the present medication on guinea pigs with citric acid-induced cough

### 1.1. Experimental materials

### 1.1.1. Medications:

(1) The present medication, 10.4 g crude drugs/g (that is 1 g of the medication is made from 10.4 g crude drugs), provided by Shijiazhuang Yiling Pharmaceutical Co., Ltd. lot number: 060401, which was prepared into the required concentrations with distilled water for the experiment.
(2) Cloperastine Hydrochloride Tablet (TABELLAE CLOPERASTINI HYDROCHLORIDI), 10mg/tablet, produced by Beijing Shuguang Pharmaceutical Co., Ltd. lot number: 060619.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 1.1.2. Animals:

Sixty purebred guinea pigs of Grade I evenly composed of males and females with the mean body weight of 238.57 ± 12.14g, provided by Beijing Keyu Animal Breeding Center, License number: SCXK (Jing) 2002-0005.

### 1.1.3. Reagent:

Citric acid, produced by Beijing Chemical Plant, lot number: 960904.

### 1.1.4 Apparatuses:

Air pump; 4 L bell-shape glass container; and glass sparger.

### 1.2. Experimental process

Prior to the experiment, the guinea pigs were individually placed into the sealed 4 L bell-shape container and sprayed for 1 minute with 17.5% of citric acid at a pressure of 400 mmHg through the glass sparger. The guinea pigs were screened with respect to cough number recorded within 5 minutes. The guinea pigs with a cough number of less than 10 were abandoned and the qualified ones were kept for the experiment.

Sixty of the screened qualified guinea pigs evenly composed of males and females were randomly assigned into the following six groups: (1) the blank group, administered with physiological saline (5 ml/kg); (2) the Cloperastine Hydrochloride Tablet group (CHT group, 7 mg/kg); (3) the Jizhi Syrup Oral Liquor group (JSOL group, 9 ml/kg); (4) the high dosage group of the present medication (H-dosage group, 6 g crude drug/kg); (5) the medium dosage group of the present medication (M-dosage group, 3 g crude drug/kg); (6) the low dosage group of the present medication (L-dosage group, 1.5 g crude drug/kg). Each animal was intragastrically administered q.d. for consecutive three days. The guinea pigs were individually placed into the sealed 4 L bell-shape container 30 min post the final administration and sprayed for 1 minute with 17.5% of citric acid at a pressure of 600 mmHg through the glass sparger. The guinea pigs were observed to record the cough incubation period and the cough number within 5 minutes. Comparisons among the groups were performed by *t*-test.

### 1.3. Test results

The experimental results show that the high, medium, and low dosages of the present medication can significantly prolong the cough incubation period and reducing the cough number and show significant difference as compared to the blank group (P<0.05, P<0.01).

**Table 1: The cough-checking effect of the present medication on guinea pig with citric acid-induced cough**

| Group | Number of animals | Dosage | Incubation period (sec) | Cough number (times /5 min) |
|---|---|---|---|---|
| | (n) | (/kg) | (*X̅±SD)* | (*X̅*±SD) |
| Blank group | 10 | 5 ml | 79.10±39.98 | 14.30±5.21 |
| CHT group | 10 | 7 mg | 173.30±68.01** | 6.20±2.04*** |
| JSOL group | 10 | 9 ml | 128.50±28.76** | 7.70±1.34** |
| H-dosage group | 10 | 6 g | 166.70±85.32** | 6.70±3.92** |
| M-dosage group | 10 | 3 g | 118.50±37.27* | 7.70±4.79** |
| L-dosage group | 10 | 1.5 g | 106.50±40.61 | 9.90±2.02* |

| | | | | |
|---|---|---|---|---|
| Note: *P<0.05, **P<0.01, ***P<0.001, as compared to the blank group. | | | | |

### 1.4. Summary

The results above show that the present medication has effects of significantly prolonging the incubation period and reducing the cough number (P<0.05, P<0.01) of the cough induced by citric acid in guinea pigs.

### 2. The cough-checking effect of the present medication on mice with an aqueous ammonia-induced cough.

### 2.1. Experimental materials

### 2.1.1. Medications:

(1) The present medication, 10.4 g crude drugs/g, provided by Shijiazhuang Yiling Pharmaceutical Co., Ltd. lot number: 060401, and prepared into the required concentrations with distilled water for the experiment.
(2) Cloperastine Hydrochloride Tablet, 10mg/tablet, produced by Beijing Shuguang Pharmaceutical Co., Ltd. lot number: 060619.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 2.1.2. Animals:

Sixty of the Kunming mice of Grade II evenly composed of males and females with the mean body weight of 19.92 ± 0.79g provided by Institute of laboratory Animal Science, Chinese Academy of Medical Sciences, License number: SCXK (Jing) 2000-0006.

### 2.1.3. Reagent: (1) aqueous ammonia, produced by Beijing Century Red Star Chemical Co., Ltd. lot number: 20060310.

### 2.2. Experiment process

Sixty of the healthy mice were randomly assigned into the following six groups: (1) the blank group administered with physiological saline (25 ml/kg); (2) the Cloperastine Hydrochloride Tablet group (CHT group, 12 mg/kg); (3) the Jizhi Syrup Oral Liquor group (JSOL group, 16 ml/kg); (4) the high dosage group of the present medication (H-dosage group, 12 g crude drug/kg); (5) the medium dosage group of the present medication (M-dosage group, 6 g crude drug/kg); (6) the low dosage group of the present medication (L-dosage group, 3 g crude drug/kg). Each mouse was subjected to intragastrical administeration q.d. for consecutive three days. 30 min post the final administration, each mouse was put into a 500 ml beaker with a tampon inside. The, 0.2 ml of aqueous ammonia was sucked into a 1 ml syringe and injected into the tampon. Then, the beaker was inverted quickly. The mouse was observed to record the cough incubation period and the cough number within 3 minutes. Comparisons among the groups were performed by *t*-test.

### 2.3. Test results

It can be seen in Table 2 that for the cough induced by aqueous ammonia in mice, the high, medium, and low dosages of the present medication can significantly prolong the incubation period of the cough and reducing the cough number and show significant difference as compared to the blank group (P<0.05- P<0.01).

**Table 2: The cough-checking effect of the present medication on mice with an aqueous ammonia-induced cough.**

| Group | Number of animals | Dosage | Incubation period (sec) | Cough number (times /5 min) |
|---|---|---|---|---|
| | (n) | (/kg) | (*X̅*±SD) | (*X̅*±SD) |
| Blank group | 0 | 25ml | 51.70±15.03 | 56.90±9.52 |
| CHT group | 10 | 12mg | 104.4±26.86*** | 31.30±9.82*** |
| JSOL group | 10 | 16ml | 92.20±29.05** | 39.80±7.60*** |
| H-dosage group | 10 | 12g | 94.50±24.67*** | 39.10±10.93** |
| M-dosage group | 10 | 6g | 72.00±8.07** | 40.40±11.31** |
| L-dosage group | 10 | 3g | 60.10±11.70 | 46.10±10.07* |

| | | | | |
|---|---|---|---|---|
| Note: ^{*}P<0.05, ^{**}P<0.01, ^{***}P<0.001, as compared to the blank group. | | | | |

### 2.4. Summary:

The results above show that the present medication has effects of significantly prolonging the incubation period of the cough and reducing the cough number (P<0.05, P<0.01, P<0.001) of the cough induced by aqueous ammonia in mice.

### 3. Conclusion:

The two experimental investigations described above suggest that the present medication has an effect for checking cough.

### Test Example 2 Experiment on the phlegm-dispelling effect of the present medication

### 1. The effect of the present medication on the amount of excreted sputum collected by capillary in rats.

### 1.1. Experimental materials

### 1.1.1. Medications:

(1) The present medication, 10.4 g crude drugs/g, provided by Shijiazhuang Yiling Pharmaceutical Co., Ltd. lot number: 060401, and prepared into the required concentrations with distilled water for the experiment.
(2) Mucosolvan, 30mg/tablet, the product of Boehringer Ingelheim Int. Ltd. German, distributed by Shanghai Sine Pharmaceutical Co. Ltd., lot number: 205325, distribution lot number:030307.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 1.1.2. Animals:

Sixty of SPF SD rats evenly composed of males and females with the mean body weight of 162.70 ± 8.25 g, provided by Vital River Laboratory Animal Technology Co., Ltd., Beijing, License number: SCXK (Jing) 2002-0003.

### 1.2 Experiment process

Sixty of the healthy rats were randomly assigned into the following six groups: (1) the blank group administered with physiological saline (10 ml/kg); (2) the Mucosolvan group (11 mg /kg); (3) the Jizhi Syrup Oral Liquor group (JSOL group, 11 ml/kg); (4) the high dosage group of the present medication (H-dosage group, 8 g crude drug/kg); (5) the medium dosage group of the present medication (M-dosage group, 4 g crude drug/kg); (6) the low dosage group of the present medication (L-dosage group, 2 g crude drug/kg). Each rat was anesthetized intraperitoneally with 3.5% of chloral hydrate (10ml/kg), laid down on its back horizontally and fixed. The skin of neck was incised and the trachea was separated. An orifice was pricked with a syringe needle at the *median site* of the lower edge of the thyroid between the two cartilages. A glass capillary was inserted through the orifice such that the glass capillary was just contact the inner wall of the trachea. When the glass capillary was filled up with sputum, it would be replaced immediately with another one. The sputum volume in capillaries was determined 1 hour prior to administration. One hour later, the animal was administered duodenally with medication (the animals in the blank control group were administered with equivalent amount of physiological saline). The average amount of secreted sputum per hour in 1 or 2 hours after administration was observed. Comparisons among the groups were performed by *t*-test with the average amount of secreted sputum 1 hour prior to administration serving as the normal value.

### 1.3. Test results

It can be seen in Table 3 that the high, medium, and low dosages of the present medication can significantly increase the secretion of the respiratory track and enhance excretion of sputum and show significant difference as compared to the blank group (P<0.05, P<0.01, P<0.001).

**Table 3: The effect of the present medication on the amount of excreted sputum measured by capillary in rats (X̅±SD, n=10).**

| Group | Dosage | Secretion (cm) | | |
|---|---|---|---|---|
| | (/kg) | 1 h prior to administration | 1 h post administration | 2 h post administration |
| Blank group | 10ml | 2.73±0.48 | 3.00±0.55^{###} | 2.87±0.56^{###} |
| | | Difference | 0.27±0.18 | 0.14±0.13 |
| | | Change rate | 1.10±0.07 | 1.05±0.05 |
| Mucosolvan group | 11mg | 2.79±0.74 | 7.03±0.90^{###} | 8.07±1.22^{###} |
| | | Difference | 4.24±0.54*** | 5.28±0.93*** |
| | | Change rate | 2.62±0.46^{ΔΔΔ} | 3.01±0.64^{ΔΔΔ} |
| JSOL group | 11 ml | 2.72±0.43 | 3.26±0.40^{###} | 3.07±0.36^{###} |
| | | Difference | 0.54±0.26* | 0.35±0.23* |
| | | Change rate | 1.21±0.12^{Δ} | 1.13±0.11^{Δ} |
| H-dosage group | 8g | 2.78±0.42 | 4.07±0.42^{###} | 3.76±0.59^{###} |
| | | Difference | 1.29±0.58*** | 0.98±0.63*** |
| | | Change rate | 1.50±0.31^{ΔΔΔ} | 1.38±0.28^{ΔΔ} |
| M-dosage group | 4g | 2.77±0.33 | 3.86±0.50^{###} | 3.44±0.51^{**} |
| | | Difference | 1.09±0.64*** | 0.67±0.60* |
| | | Change rate | 1.41±0.27^{ΔΔ} | 1.26±0.24^{Δ} |
| L-dosage group | 2g | 2.78±0.32 | 3.28±0.29^{###} | 3.09±0.25^{###} |
| | | Difference | 0.50±0.21* | 0.31 ±0.23 |
| | | Change rate | 1.19±0.09^{Δ} | 1.12±0.09 |

| | | | | |
|---|---|---|---|---|
| Note: ^{*}P<0.05, ^{***}P<0.001, as compared to the blank group; ^{*Δ}P<0.0015; ^{ΔΔ}P<0.01; ^{ΔΔΔ}P<0.001, as compared to the blank group; ^{##}P<0.01, ^{###}P<0.001, as self-compared. | | | | |

### 1.4. Summary

The above-described results suggest that the present medication has an effect of increasing the excreted sputum volume (P<0.05, P<0.01, P<0.001).

### 2. Effect of the present medication on dispelling phlegm measured by phenol red in mice.

### 2.1. Experimental materials

### 2.1.1. Medications:

(1) The present medication, 10.4 g crude drugs/g, provided by Shijiazhuang Yiling Pharmaceutical Co. Ltd., lot number: 060401, and prepared into the required concentrations with distilled water for the experiment.
(2) Mucosolvan, 30mg/tablet, the product of Boehringer Ingelheim lnt. Ltd. German, distributed by Shanghai Sine Pharmaceutical Co.Ltd., lot number: 205325, distribution lot number:030307.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 2.1.2. Reagent:

Phenol red (an indicator) from Beijing Chemical Plant, lot number: 770517.

### 2.1.3. Animals:

Sixty of the ICR mice of Grade 11 evenly composed of males and females with the mean body weight of 19.30 ± 1.15 g, provided by Vital River Laboratory Animal Technology Co., Ltd., Beijing, License number: SCXK (Jing) 2002-0003.

### 2.2. Experiment process

Sixty of the healthy mice were randomly assigned into the following six groups: (1) the blank group administered with physiological saline (25 ml/kg); (2) the Mucosolvan group (16 mg/kg); (3) the Jizhi Syrup Oral Liquor group (JSOL group, 16 ml/kg); (4) the high dosage group of the present medication (H-dosage group, 12 g crude drug/kg); (5) the medium dosage group of the present medication (M-dosage group, 6 g crude drug/kg); (6) the low dosage group of the present medication (L-dosage group, 3 g crude drug/kg). Each mouse was intragastrically administered q.d. for consecutive three days, while being fasted but having *Ad libitum* access to water prior to the experiment. After the final administration, the mouse was injected with a solution of 5% phenol red physiological saline at 500mg/kg. Thirty minutes later, the mouse was sacrificed and fixed in dorsal position. The skin of neck was incised medianly and the trachea was separated. A tiny opening was cut on the separated trachea and an obtuse syringe needle of 7 Gauge was inserted about 0.3 cm into the trachea through the opening and then ligated firmly with a silk suture. The airway was washed repeatedly with 0.5 ml of physiological saline sucked into a 1 ml syringe and the flush liquid was aspirated into a test tube. Those above said steps were repeated 3 times. 0.1ml of 1 mol/L NaOH was added to the flush liquid in the tube, allowing the liquid to be basic. The optical density (OD) value in the flush liquid was determined by UV-visible spectrophotometer UV-120-02 at a wavelength of 546nm, in which the level of phenol red in the flush liquid (µg/ml) was calculated on basis of a phenol red standard curve. Comparisons among the groups were performed by *t-*test.

### 2.3. Test results

The results in Table 3 show that both the high and medium dosages of the present medication have an effect of significantly increasing the amount of phenol red excreted by mucus membrane of respiratory tract in mice and show significant difference as compared to the blank group. The results suggest that the present medication has an apparent effect of dispelling phlegm (P<0.05, P<0.001).

**Table 4: Effect of the present medication on phenol red expectoration in the mouse (X̅±SD n=10)**

| Group | Dosage (/kg) | Amount of phenol red excreted (µg/ml) |
|---|---|---|
| Blank group | 25ml | 0.507±0.105 |
| Mucosolvan group | 16mg | 1.538±0.486^{***} |
| JSOL group | 16ml | 0.683±0.159 |
| H-dosage group | 12g | 0.943±0.320^{***} |
| M-dosage group | 6g | 0.889±0.461^{*} |
| L-dosage group | 3g | 0.618±0.145 |

| | | |
|---|---|---|
| Note: ^{*}P<0.05, ^{***}P<0.001, as compared to the control group. | | |

### 2.4. Summary

The above-described result suggests that the high and medium dosages of the present medication have an effect of increasing the amount of phenol red excreted through the respiratory tract of the mouse, thus exerting a significant phlegm-dispelling effect on the mouse (P<0.05, P<0.001).

### 3 Conclusion:

The two experimental investigations described above suggest that the present medication has an effect of dispelling phlegm.

### Test example 3 Experiment on wheezing asthma-relieving effect of the present medication

### 1. Wheezing asthma-relieving effect of the present medication on histamine-induced asthma in guinea pigs

### 1.1. Experimental materials

### 1.1.1. Medications:

(1) The present medication, 10.4 g crude drugs/g, provided by Shijiazhuang Yiling Pharmaceutical Co. Ltd., lot number: 060401, and prepared into the required concentrations with distilled water for the experiment.
(2) Aminophylline, 0.1g/tablet, from Beijing Zizhu Pharmaceutical Co., Ltd, lot number: 20061202.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 1.1.2. Animals:

Sixty of white purebred guinea pigs of Grade I evenly composed of males and females with the mean body weight of 181.97 ± 15.85 g, provided by Beijing Keyu Animal Breeding Center, License number: SCXK (Jing) 2002-0005.

### 1.1.3. Reagent:

(1) Acetylcholine chloride, 1g/bottle, from Beijing Chemical Reagents Company, lot number 060211.
(2) Histamine phosphate, 5g/bottle, from Shanghai Institutes of Biology, Chinese Academy of Sciences, production No. 1702.

### 1.1.4. Instrument:

An asthma-inducing apparatus (air compressor, glass nebulizer), 4L bell-shape glass container.

### 1.2. Experiment process

Healthy guinea pigs evenly composed of males and females were subjected to screening previously. The guinea pigs were individually placed into the 4 L bell shape container and sprayed with a mixed liquid of 2 % acetylcholine chloride and 0.1 % histamine phosphate in equal volumes for 20 s at a pressure of 400 mmHg. After the spraying, the guinea pigs were observed for the incubation period for asthmatic convulsion (the time that the asthmatic convulsion takes to occur after ceasing the spaying) within 6 minutes. Guinea pigs showing the incubation period for asthmatic convulsion of above 120 s would not be chosen.

Sixty of the screened guinea pigs were randomly assigned into the following six groups: (1) the control model group administered with physiological saline (5 ml/kg); (2) the Aminophylline group (0.05 g/kg); (3) the Jizhi Syrup Oral Liquor group (JSOL group, 9 ml/kg); (4) the high dosage group of the present medication (H-dosage group, 6 g crude drug/kg); (5) the medium dosage group of the present medication (M-dosage group, 3 g crude drug/kg); (6) the low dosage group of the present medication (L-dosage group, 1.5 g crude drug/kg). The animals were intragastrically administered q.d. for consecutive three days and subjected to the asthma-inducing experiment in 30 min post the final administration. The guinea pigs were individually placed into the 4 L bell-shape glass container and sprayed with a mixed liquid of 2 % acetylcholine chloride and 0.1% histamine phosphate in equal volumes for 20 s at a pressure of 400 mmHg. Then, the guinea pigs were observed within 6 minutes for the incubation period of asthmatic convulsion (also known as the asthma-inducing incubation period), that is, the time it takes for asthmatic convulsion to occur and for the guinea pig to fall down after ceasing the spaying. The incubation period lasting for more than 6 min was counted as 360 s. The experimental results were compared among the groups by *t*-test.

### 1.3. Test results

**Table 5: Wheezing asthma-relieving effect of the present medication on guinea pigs (n=10, X̅±SD).**

| Group | Dosage | Incubation period | Falling-down time |
|---|---|---|---|
| | (g /kg) | (second) | (second) |
| Control model group | 5ml | 67.20±44.32 | 129.40±49.46 |
| Aminophylline group | 0.05g | 228.0±43.35^{***} | 358.50±3.37^{***} |
| JSOL group | 9ml | 123.30±37.04^{**} | 215.10±110.21^{*} |
| H-dosage group | 6g | 124.40±30.12^{**} | 231.20±94.82^{**} |
| M-dosage group | 3g | 110.70±41.39^{*} | 211.40±105.13^{*} |
| L-dosage group | 1.5g | 93.20±50.65 | 160.20±49.40 |

| | | | |
|---|---|---|---|
| Note: ^{*}P<0.05; ^{**}P<0.01; ^{***}P<0.001, as compared to the control model group. | | | |

It can be seen from the results in Table 5 that the high and medium dosages of the present medication have an effect of significantly prolonging the asthma incubation period and deferring the fall-down as compared to the control group (P<0.05, P<0.01), while the low dosage can somewhat prolong the incubation period and defer the fall-down as compared to the control group, but displays no significant difference.

### 1.4. Summary

The experimental results show that the high and medium dosages of the present medication has an effect of significantly prolonging the asthma incubation period and deferring the fall-down, thus significantly ameliorating the histamine-induced asthma in guinea pigs (P<0.05, P<0.01).

### 2. Effect of the present medication on allergic bronchial spasm in guinea pigs

### 2.1. Experimental materials

### 2.1.1. Medications:

(1) The present medication, 10.4 g crude drugs/g, provided by Shijiazhuang Yiling Pharmaceutical Co. Ltd., lot number: 060401, and prepared into the required concentrations with distilled water for the experiment.
(2) Aminophylline, 0.1g/tablet, the product from Beijing Zizhu Pharmaceutical Co., Ltd, lot number: 20061202.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 2.1.2. Animals:

Sixty of white purebred guinea pigs of Grade I evenly composed of males and females with the mean body weight of 188.15 ± 16.07 g, provided by Beijing Keyu Animal Breeding Center, License number: SCXK (Jing) 2002-0005.

### 2.1.3. Antigen:

Ovalbumin, 50g/bottle, from Sigma, Cat: A5253, distributed by Beijing Hui Ze Ao Technology and Trade Co., Ltd.

### 2.1.4. Reagent:

Pertussis vaccine, thirty billion units/ml, 2ml/ampule, from National Institute For Control of Pharmaceutical and Biological Products, lot number: 04-1.

### 2.1.5. Instrument

An asthma-inducing apparatus, (air compressor, glass nebulizer), a 4L bell-shape glass container.

### 2.2. Experiment process

Sensitization of the guinea pigs: sixty of the white healthy guinea pigs were selected and each of them was injected intramuscularly at the rear leg with 4 mg of ovalbumin (0.1 ml of physiological saline of 4% ovalbumin) and at same time injected intraperitoneally with 2×10¹⁰ bacteria of the pertussis vaccine. After sensitization for 14 days, the injected animals were used in the experiment.

The animals at Day 10 after the sensitization were randomly assigned into the following six groups with 10 animals per group: (1) the control group administered with physiological saline (5 ml/kg); (2) the Aminophylline group (0.05 g/kg); (3) the Jizhi Syrup Oral Liquor group (JSOL group, 9 ml/kg); (4) the high dosage group of the present medication (H-dosage group, 6 g crude drug/kg); (5) the medium dosage group of the present medication (M-dosage group, 3 g crude drug/kg); (6) the low dosage group of the present medication (L-dosage group, 1.5 g crude drug/kg), and intragastrically administered q.d. for consecutive five days. The guinea pigs were individually put into the sealed 4L bell shape container in 30 min post the final administration and sprayed with a solution of 5 % ovalbumin for half of a minute at the constant pressure of 400 mmHg. The guinea pigs were observed to record the incubation period of asthmatic convulsion and the number of animals appearing asthma, convulsionary fall-down as well as death within 6 minutes. As for the animal not showing asthma, convulsionary fall-down within 6 minutes, the incubation period was counted as 360 s. The experimental results were compared among the groups by *t*-test. The numbers of the dead animals were statistically test by Chi-square X² test.

### 2.3. Test results

**Table 6: Asthma-relieving effect of the present medication on guinea pigs (n=10, X̅±SD).**

| Group | Dosage | Incubation period | Asthma | Convulsionary fall-down | Number of the dead animals |
|---|---|---|---|---|---|
| | (g/kg) | (sec) | (sec) | (sec) | (sec) |
| Control group | 5ml | 39.70±10.79 | 64.40±21.61 | 87.90±29.86 | 9 |
| Aminophylline group | 0.05g | 330.00±53.28*** | 387.00±83.73*** | 352.50±23.72*** | 0*** |
| JSOL group | 9ml | 59.50±25.87* | 182.00±129.77* | 206.50±136.28* | 4* |
| H-dosage group | 6g | 70.70±31.455** | 164.70±107.05** | 209.40±132.50* | 2** |
| M-dosage group | 3g | 63.10±31.11* | 188.70±147.94* | 196.00±141.87* | 5* |
| L-dosage group | 1.5g | 42.50±113.79 | 125.70±123.99 | 133.70±120.77 | 7 |

| | | | | | |
|---|---|---|---|---|---|
| Note: ^{*}P<0.05; ^{**}P<0.01; ^{***}P<0.001, as compared to the control group. | | | | | |

It can be seen from the results in Table 6 that the high and medium dosages of the present medication can significantly prolong the incubation period of allergic bronchial spasm, defer the occurrence of asthma and convulsionary fall-down, and reduce the number of the dead animals, and show significant differences as compared to the control group (P<0.05, P<0.01), while the low dosage can somewhat prolong the incubation period of allergic bronchial spasm and defer the occurrence of asthma and convulsionary fall-down as compared to the control group, but shows no significant difference.

### 2.4. Summary

The experimental results show that the high and medium dosages of the present medication have effects of prolonging the asthma incubation period, deferring the occurrence of asthma and convulsionary fall-down, and reducing the number of the dead animals, thus significantly ameliorating the ovalbumin-induced allergic bronchial spasm (P<0.05, P<0.01).

### 3. Conclusion:

The two experimental investigations described above suggest that the present medication has an effect of relieving asthma.

### Test Example 4 Experiment on pyretotysis effect of the present medication

### 1. Effect of the present medication on Escherichia coli endotoxin-induced pyrexia in rabbits.

### 1.1. Experimental materials

### 1.1.1. Medications:

(1) The present medication, 10.4 g crude drugs/g, provided by Shijiazhuang Yiling Pharmaceutical Co. Ltd., lot number: 060401, and prepared into the required concentrations with distilled water for the experiment.
(2) Aspirin enteric-coated tablet, 0.3 g/tablet, produced by Yantai Justaware Pharmaceutical Co., Ltd. (Yantai Second Pharmaceutical Factory), lot number: 061102.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product 0611034010.

### 1.1.2. Pyrogen:

*E. coli.* endotoxin, 500 µg/ampule, from Institute of Biological products, Ministry of Health of the People's Republic of China, lot number: 891-2.

### 2.1.3. Animals:

Forty-eight of the Japanese White rabbits evenly composed of males and females with the mean body weight of 2.10 ± 0.08 kg, provided by Tongli Laboratory Animal Farm, Xueyuan Road, Haidian District, Beijing; Qualified certificate NO: License number SCK (Jing) 2002-0008.

### 1.2. Experiment process

The Japanese White rabbits were assigned into the following six groups with eight animals per group, evenly composing of males and females: (1) the control model group administered with physiological saline (3 ml/kg); (2) the Aspirin group (100 mg/kg); (3) the Jizhi Syrup Oral Liquor group (JSOL group, 6 ml/kg); (4) the high dosage group of the present medication (H-dosage group, 4 g crude drug/kg); (5) the medium dosage group of the present medication (M-dosage group, 2 g crude drug/kg); (6) the low dosage group of the present medication (L-dosage group, 1 g crude drug/kg). Each rabbit was indued fever by *E. coli.* endotoxin and was measured the rectal temperature twice at the day immediately before the experiment. The average temperature was used as the body temperature prior to administration. The next day, the rabbit was subjected to the intragastrical administration q.d. for consecutive three days (the animals in the control model group were intragastrically administered with the equal volume of physiological saline, and the animals in the Aspirin group were intragastrically administered once before injected with the endotoxin). The animals in the administered groups were injected with *E. coli.* endotoxin at 250 ng/ml/kg through the marginal ear vein 30 minute post the final administration, the body temperatures were determined at 1, 2, 3, 4, 5, and 6 hours post the injection, and changes in the body temperatures at each of the time points (Δ °C) were calculated. Comparisons among the groups were performed by *t*-test.

### 1.3. Test results (see Table 7)

**Table 7: Effect of the present medication on rabbit's body temperature (n=8, X̅±SD)**

| Group | Dosage (/kg) | Prior to administration (T, °C) | Changes of body temperature post final administration (Δ °C) | | | | | |
|---|---|---|---|---|---|---|---|---|
| | | | 1h | 2h | 3h | 4h | 5h | 6h |
| Control group | 3ml | 39.25± 0.20 | 40.10± 0.43^{###} | 40.49± 0.34^{###} | 40.80± 0.27^{###} | 41.00± 0.37^{###} | 40.24± 0.37^{###} | 39.79 ±0.36^{##} |
| | | Difference | 0.85± 0.41 | 1.24± 0.39 | 1.55± 0.35 | 1.75± 0.44 | 0.99± 0.38 | 0.54± 0.38 |
| Aspirin group | 100mg | 39.28± 0.24 | 39.58± 0.13^{###} | 39.81± 0.29^{###} | 39.66± 0.56 | 39.26± 0.52 | 39.04± 0.49 | 38.85± 0.52 |
| | | Difference | 0.30± 0.15** | 0.54± 0.30*** | 0.39± 0.59*** | -0.01± 0.52*** | -0.24± 0.51*** | -0.42± 0.53*** |
| JSOL group | 6g | 39.13±0.36 | 39.54± 0.45^{###} | 39.90± 0.52^{###} | 40.19± 0.55^{###} | 40.41± 0.50^{###} | 40.09± 0.57^{###} | 39.61± 0.38^{##} |
| | | Difference | 0.41± 0.21* | 0.77± 0.37* | 1.06± 0.40* | 1.29± 0.39* | 0.96± 0.39 | 0.49± 0.36 |
| H-dosage group | 4g | 39.14±0.14 | 39.63± 0.21^{###} | 39.91± 0.22^{###} | 40.24± 0.24^{###} | 40.44± 0.26^{###} | 39.69± 0.36^{##} | 39.26± 0.33 |
| | | Difference | 0.49± 0.17* | 0.77± 0.19** | 1.10± 0.21** | 1.30± 0.28* | 0.55± 0.41* | 0.13± 0.38* |
| M-dosage group | 2g | 39.28±0.17 | 39.75± 0.09^{###} | 40.13± 0.19^{###} | 40.44± 0.20^{###} | 40.60± 0.15^{###} | 39.88± 0.42 | 39.44± 0.39 |
| | | Difference | 0.48± 0.16^{*} | 0.85t 0.30^{*} | 1.16± 0.29^{*} | 1.33± 0.29^{*} | 0.60± 0.51 | 0.16± 0.48 |
| L-dosage group | 1g | 39.25±0.27 | 39.96± 0.18^{###} | 40.45± 0.18^{###} | 40.71± 0.24^{###} | 40.79± 0.19^{###} | 40.14± 0.35^{##} | 39.69± 0.34^{*} |
| | | Difference | 0.71±0.30 | 1.20±0.29 | 1.46±0.31 | 1.54±0.36 | 0.89±0.49 | 0.44±0.36 |

| | | | | | | | | |
|---|---|---|---|---|---|---|---|---|
| Note: ^{*}P<0.05, ^{**}P<0.01, as compared to the control group. ^{#}P<0.05; ^{##}P<0.01; ^{###}P<0.001, as self-compared. | | | | | | | | |

It can be seen from the results in Table 7 , the body temperatures of the animals in the control model group began to rise in 1 h post pyretogenesis, reached peak in 4 h post pyretogenesis, and decreased gradually in 6 h post pyretogenesis. Pyretolysis in the Aaspirin group was obvious and has significant difference as compared to the control model group in 1-6 hours post pyretogenesis (P<0.01 - P<0.001). Pyretolysis in the high dosage group was apparent in 1-6 hours post pyretogenesis , even more apparent in 2-3 hours post pyretogenesis, and has significant difference as compared to the control model group (P<0.05 - P<0.01). Pyretolysis in the medium dosage group has significant difference as compared to the control model group in 1-4 hours post pyretogenesis (P<0.05 - P<0.01). Pyretolysis in the low dosage group was not apparent.

### 1.4. Summary

The high and medium dosages of the present medication exert an obvious heat-relieving effect on the pyrexial rabbit model induced by *E. coli.* endotoxin

### 2. Effect of the present medication on yeast-induced pyrexia in rats

### 2.1. Experimental materials

### 2.1.1. Medications:

(1) The present medication, 10.4 g crude drugs/g, provided by Shijiazhuang Yiling Pharmaceutical Co. Ltd., lot number: 060401, and prepared into the required concentrations with distilled water for the experiment.
(2) Aspirin enteric-coated tablet, 0.3 g/tablet, produced by Yantai JUSTAWARE Pharmaceutical Co., Ltd. (Yantai the Second Pharmaceutical Factory), lot number: 061102.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 2.1.2. Pyrogen:

Dried yeast tablet, 0.2g/tablet, produced by Zhanjiang Wuzhou Pharmaceutcal Ltd. (previously known as Zhuxi Count Pharmaceutical Factory, Guangdong Province), lot 38 number: 0611237.

### 2.1.3. Animals:

Sixty of the SPF SD rats evenly composed of males and females with the mean body weight of 178.27 ± 10.42 g, provided by Vital River Laboratory Animal Technology Co., Ltd., Beijing, License number. SCXK (Jing) 2002-0003.

### 2.2. Experiment process

The animals were assigned randomly into the following six groups with ten animals per group, evenly composing of males and females. (1) the control model group administered with physiological saline (10 ml/kg); (2) the Aspirin group (0.15 g/kg); (3) the Jizhi Syrup Oral Liquor group (JSOL group, 11 ml/kg); (4) the high dosage group of the present medication (H-dosage group, 8 g crude drug/kg); (5) the medium dosage group of the present medication (M-dosage group, 4 g crude drug/kg); (6) the low dosage group of the present medication (L-dosage group, 2 g crude drug/kg). The rectal temperatures of the rats were measured twice at the day immediately before the experiment. The average temperature was used as the body temperature prior to administration. The next day, each animal was subjected tointragastrical administration q.d. for consecutive three days (the animals in the control model group were intragastrically administered with equal volume of physiological saline). The rats in the administered group were subcutaneously injected with a suspension of 15% yeast (10ml/kg) in 30 min post the final administration, the rats in the aspirin model group were intragastrically administered once before injection of the yeast suspension. The body temperatures of the rats in the administered groups were determined at 2, 3, 4, 5, 6, 7, 8, and 9 hours post the injection. The rats in the administered group were again intragastrically administered (with the same dosage as the aforementioned). Changes in the body temperatures at each of the time points (Δ °C) were calculated. Comparisons among the groups were performed by *t*-test.

### 2.3. Test results

It can be seen from Table 8 , the body temperatures of the animals in the control model groups began to rise in 2 h post pyretogenesis, reached the peak in 5 h post pyretogenesis, and decreased gradually in 6 h post pyretogenesis. Pyretolysis in the aspirin group was obvious and has significant difference as compared to the control model group in 2-9 hours post pyretogenesis (P<0.001). Pyretolysis in the high dosage group was apparent in 2-9 hours post pyretogenesis, even more apparent in 3-5 hours post pyretogenesis, and has significant difference as compared to the control model group (P<0.05, P<0.01). Pyretolysis in the mdeium dosage group has significant difference as compared to the control model group in 2-5 hours post pyretogenesis (P<0.05 - P<0.01). Pyretolysis in the low dosage group was not apparent. The experimental results show that the high and medium dosages of the present medication have a significant effect of relieving the yeast-induced pyrexia in rats.

**Table 8: Effect of the present medication on rat's body temperature (n=8, X̅±SD)**

| Group | Dosage (/kg) | Prior to administer (T, °C) | Changes In the body temperature post the final administration (Δ °C) | | | | | | | |
|---|---|---|---|---|---|---|---|---|---|---|
| | | | 2h | 3h | 4h | 5h | 6h | 7h | 8h | 9h |
| Control model group | 10ml | 38.88± 0.20 | 39.19± 0.21^{###} | 40.05± 0.32^{###} | 40.23± 0.31^{###} | 40.38± 0.29^{###} | 40.25± 0.20^{###} | 40.14± 0.25^{###} | 39.99± 0.22^{###} | 39.65 ±0.20^{###} |
| | | Difference | 0.31± 0.10 | 1.17± 0.36 | 1.35± 0.34 | 1.50± 0.32 | 1.37± 0.27 | 1.26± 0.26 | 1.11± 0.29 | 0.77± 0.31 |
| Aspirin group | 0.15g | 38.84± 0.12 | 38.88±: 0.12 | 39.11± 0.34^{#} | 39.19± 0.31^{1##} | 39.06± 0.26^{#} | 38.93± 0.21 | 38.82± 0.20 | 38.62± 0.21^{#} | 38.51± 0.21^{##} |
| | | Difference | 0.04± 0.10*** | 0.27±0.31*** | 0.35± 0.28*** | 0.22± 0.24*** | 0.09± 0.20*** | -0.02± 0.20*** | -0.22± 0.25*** | -0.33± 0.25*** |
| JSOL group | 11ml | 38.88± 0.17 | 38.98± 0.19 | 39.68± 0.20^{###} | 39.81± 0.19^{###} | 40.03± 0.16^{###} | 39.96± 0.20^{###} | 39.93± 0.12^{###} | 39.81± 0.23^{###} | 39.68± 0.18^{###} |
| | | Difference | 0.10± 0.24* | 0.80± 0.16** | 0.93± 0.28** | 1.15± 0.25* | 1.08± 0.29* | 1.05± 0.21 | 0.93± 0.23 | 0.80± 0.16 |
| H-dosage group | 8g | 38.91± 0.19 | 39.09± 0.20" | 39.52± 0.21^{###} | 39.74± 0.20^{###} | 39.92± 0.18^{###} | 39.93± 0.21^{###} | 39.83± 0.19^{###} | 39.69± 0.20^{###} | 39.33± 0.27^{##} |
| | | Difference | 0.18± 0.13* | 0.61± 0.30** | 0.83± 0.25*** | 1.01± 0.24** | 1.02± 0.28* | 0.92± 0.27* | 0.78± 0.27* | 0.42± 0.24* |
| M-dosag e group | 4g | 38.82± 0.27 | 39.01± 0.21^{###} | 39.56± 0.21^{###} | 39.78± 0.17^{###} | 39.99± 0.17^{###} | 39.91± 0.28^{###} | 39.82± 0.25^{###} | 39.77± 0.25^{###} | 39.51± 0.21^{###} |
| | | Difference | 0.19± 0.10* | 0.74± 0.30*** | 0.96± 0.28* | 1.17± 0.28* | 1.09± 0.38 | 1.00± 0.30 | 0.95± 0.28 | 0.69± 0.35 |
| L-dosage group | 2g | 38.82± 0.24 | 39.05± 0.20^{###} | 39.75± 0.25^{###} | 39.91± 0.23^{###} | 40.06± 0.18^{###} | 40.09± 0.26^{###} | 39.89± 0.37^{###} | 39.76± 0.29^{###} | 39.44± 0.28^{###} |
| | | Difference | 0.23± 0.08 | 0.93± 0.29 | 1.09± 0.32 | 1.24± 0.30 | 1.27± 0.26 | 1.07± 0.28 | 0.94± 0.18 | 0.62± 0.36 |

| | | | | | | | | | | |
|---|---|---|---|---|---|---|---|---|---|---|
| Note: ^{*}P<0.05; ^{**}P<0.01; ^{***}P<0.001, as compared to the control model group. ^{#}P<0.05; ^{##}P<0.01; ^{###}P<0.001, as self-compared. | | | | | | | | | | |

### 2.4. Summary

In the experiment, the heat-relieving effect of the present medication was observed on a pyrexial rat model induced by yeast. The results suggest that the high and medium dosages of the present medication can significantly reduce the yeast-induced fever in rats, thus exert better heat-relieving effect (P<0.05, P<0.01).

### 3. Conclusion:

The two experimental investigations described above suggest that the present medication has an effect of heat- relieving.

### Test Example 5: Anti-inflammatory effect of the present medication

### 1. Effect of the present medication on carrageenan-induced swelling in rat's footpad

### 1.1. Experimental materials

### 1.1.1 Medications:

(1) The present medication, 10.4 g crude drugs/g, provided by Shijiazhuang Yiling Pharmaceutical Co. Ltd., lot number: 060401, and prepared into the required concentrations with distilled water for the experiment.
(2) Aspirin enteric-coated tablet, 0.3 g/tablet, produced by Yantai JUSTAWARE Pharmaceutical Co., Ltd. (Yantai the Second Pharmaceutical Factory), lot number: 061102.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.
(4) Carrageenan, from SIGMA, lot number: 117H0151, prepared into the required concentrations with distilled water for the experiment,

### 1.1.2. Animals:

Sixty of the Wistar male rats of Grade I with the mean body weight of 130.15± 5.79g, provided by Institute of laboratory animal science, Chinese Acadamy of Medical Sciences, License number: SCXK (Jing) 2000-0006.

### 1.2. Experiment process

Sixty of the healthy rats were randomly assigned into the following six groups: (1) the control group administered with physiological saline (10 ml/kg); (2) the Aspirin group (0.15 g /kg); (3) the Jizhi Syrup Oral Liquor group (JSOL group, 11 ml/kg); (4) the high dosage group of the present medication (H-dosage group, 8 g crude drug/kg); (5) the medium dosage group of the present medication (M-dosage group, 4 g crude drug/kg); (6) the low dosage group of the present medication (L-dosage group, 2 g crude drug/kg). The volume of the left hind foot (ml) of each animal was measured by capillary ammplification method and used as the value prior to administration. The intragastrical administration as the route of administration was performed q.d. for consecutive three days, while the animals in the control model group were intragastrically administered with the equal volume of physiological saline. Each rat was inflamed by subcutaneous injection of 1% carrageenan at 0.05 ml per animal into the rat's left footpad in 30 min post the final administration. The volumes of the rat left hind foot were determined in 0.5, 1, 2, 4, and 6 hours after inflammation. Comparisons among the groups were performed on the differences between the paw volumes before and after inflammation by *t*-test.

### 1.3. Test results

As shown in Table 9, the high dosage of the present medication can.significantly inhibit the rat foot swelling at various time points of 0.5, 1, 2, 4, and 6 hours after inflammation and shows significant difference as compared to the control group (P<0.01, P<0.001); the medium dosage of the present medication can significantly inhibit the rat foot swelling in 0.5, 1, 2, 4, and 6 hours after inflammation and shows significant difference as compared to the control group (P<0.05); and the low dosage of the present medication doesn't show apparent swelling-inhibitory effect.

**Table 9: Effect of the present medication on carrageenan-induced swelling in rat's footpad (n=10, X̅ ±SD)**

| Group | Dosage | Before inflammation | Changes in foot volume after inducing inflammation (ml) | | | | |
|---|---|---|---|---|---|---|---|
| | (/kg) | foot volume (ml) | 0.5h | 1h | 2h | 4h | 6h |
| Control model group | 10ml | 0.96±0.03 | 1.31± 0.06^{###} | 1.37± 0.03^{###} | 1.43± 0.03^{###} | 1.46± 0.02^{###} | 1.44± 0.01^{###} |
| | | Difference | 0.35± 0.07 | 0.42± 0.03 | 0.47t 0.04 | 0.511± 0.03 | 0.49± 0.03 |
| | | Swelling rate (%) | 36.93± 7.94 | 43.59± 4.36 | 49.15± 4.88 | 53.23± 4.04 | 51.77± 4.17 |
| Aspirin group | 0.15g | 0.98±0.02 | 1.19± 0.04^{###} | 1.23± 0.03^{###} | 1.28± 0.03^{###} | 1.32± 0.03^{###} | 1.32± 0.03^{###} |
| | | Difference | 0.21± 0.04*** | 0.26± 0.03*** | 0.31± 0.04*** | 0.34± 0.04*** | 0.34± 0.03*** |
| | | Swelling rate (%) | 21.55± 4.53^{ΔΔΔ} | 26.27± 3.48^{ΔΔΔ} | 31.60± 4.08^{ΔΔΔ} | 35.18± 3.98^{ΔΔΔ} | 34.57± 3.94^{ΔΔΔ} |
| | | Inhibition rate (%) | 41.66 | 39.75 | 35.70 | 33.91 | 33.23 |
| JSOL group | 11ml | 0.97±0.05 | 1.24± 0.02^{###} | 1.30± 0.04^{###} | 1.37± 0.04^{###} | 1.41± 0.03^{###} | 1.40± 0.03^{###} |
| | | Difference | 0.27± 0.04** | 0.33± 0.07** | 0.40± 0.07** | 0.44± 0.07** | 0.43± 0.07* |
| | | Swelling rate (%) | 27.66± 5.95^{ΔΔ} | 34.55± 8.40^{ΔΔ} | 41.18± 8.99^{Δ} | 45.74± 9.10^{Δ} | 44.32± 9.50^{Δ} |
| | | Inhibition rate (%) | 25.09 | 20.74 | 16.20 | 14.07 | 14.39 |
| H-dosage group | 8g | 0.97±0.05 | 1.24± 0.02^{###} | 1.29± 0.03^{###} | 1.35± 0.02^{###} | 1.41± 0.02^{###} | 1.41± 0.03^{###} |
| | | Difference | 0.26± 0.05** | 0.32± 0.05*** | 0.38± 0.05*** | 0.44± 0.03*** | 0.43± 0.03*** |
| | | Swelling rate (%) | 27.40± 6.15^{ΔΔ} | 33.16± 6.19^{ΔΔΔ} | 38.93± 6.33^{ΔΔΔ} | 45.49± 5.55^{ΔΔ} | 44.85± 5.25^{ΔΔ} |
| | | Inhibition rate (%) | 25.81 | 23.94 | 20.78 | 14.54 | 13.36 |
| M-dosage group | 4g | 0.98±0.06 | 1.25± 0.02^{###} | 1.33± 0.02^{###} | 1.39± 0.02^{###} | 1.43± 0.02^{###} | 1.44± 0.02^{###} |
| | | Difference | 0.27± 0.06* | 0.36± 0.06* | 0.42± 0.05* | 0.45± 0.06* | 0.46± 0.06 |
| | | Swelling rate (%) | 28.45± 7.60* | 37.12± 8.53* | 43.01± 7.70* | 46.94± 8.40* | 47.55± 8.50 |
| | | Inhibition rate (%) | 22.96 | 14.86 | 12.49 | 11.82 | 8.14 |
| L-dosage group | 2g | 0.98±0.05 | 1.28± 0.01^{###} | 1.36± 0.02^{###} | 1.41± 0.03^{###} | 1.45± 0.03^{###} | 1.44± 0.03^{###} |
| | | Difference | 0.30± 0.05 | 0.39± 0.05 | 0.43± 0.05 | 0.47± 0.05 | 0.47± 0.04 |
| | | Swelling rate (%) | 31.25± 6.56 | 39.86± 6.53 | 44.77± 6.70 | 48.88± 7.19 | 48.02± 6.16 |
| | | Inhibition rate (%) | 15.38 | 8.57 | 8.91 | 8.16 | 7.25 |

| | | | | | | | |
|---|---|---|---|---|---|---|---|
| Note: *P<0.05; **P<0.01; ***P<0.001, as compared to the control group. ^{###}P<0.001, as setf-compared. ^{Δ}P<0.0015; ^{ΔΔ}P<0.01; ^{ΔΔΔ}P<0.001, as compared to the control group. | | | | | | | |

### 1.4. Summary

The results suggest that the high and medium dosages of the present medication can significantly inhibit the carrageenan-induced swelling inflammation in the rat footpad (P<0.05, P<0.01, P<0.001).

### 2. Effect of the present medication on xylene-induced ear swelling in mice

### 2.1. Experimental materials

### 2.1.1. Animals:

Sixty of the SPF ICR male mice with the mean body weight of 20.35 ± 0.61 g, provided by Vital River Laboratory Animal Technology Co., Ltd., Beijing, License number. SCXK (Jing) 2002-0003.

### 2.1.2. Medications:

(1) The present medication, 10.4 g crude drugs/g, provided by Shijiazhuang Yiling Pharmaceutical Co. Ltd., lot number: 040401, and prepared into the required concentrations with distilled water for the experiment.
(2) Aspirin enteric-coated tablet, 0.3 g/tablet, produced by Yantai Justaware Pharmaceutical Co., Ltd. (Yantai the Second Pharmaceutical Factory), lot number: 061102.
(3) Jizhi Syrup Oral Liquor, 200ml/bottle, produced by Fuling Pharmaceutical Factory, Taiji Group, with approval number: SFDA Approval No. Z50020615, production date: November 13, 2006, and lot number of the product: 0611034010.

### 2.1.3. Reagent:

Xylene, A.P., produced by Beijing Chemical Plant, lot number: 060202.

### 2.2. Experiment process

Sixty of the healthy mice were randomly assigned into the following six groups with ten animals per group: (1) the control model group administered with physiological saline (25 ml/kg); (2) the Aspirin group (0.2 g/kg); (3) the Jizhi Syrup Oral Liquor group (JSOL group, 16 ml/kg); (4) the high dosage group of the present medication (H-dosage group, 12 g crude drug/kg); (5) the medium dosage group of the present medication (M-dosage group, 6 g crude drug/kg); (6) the low dosage group of the present medication (L-dosage group, 3 g crude drug/kg). Each mouse was subjected to intragastrical administration q.d. for consecutive three days, and at 30 minutes after the administration at Day 3, 0.1 ml of xylene was dropped onto the right ear of the mouse. Fifteen minutes later, the animal was sacrificed by dislocation. Holes were punched with a hole puncher of 6 mm at the identical sites on the right and left auricles. The pieces from the right and left ears were weighted respectively on an electronic balance. The degree of ear swelling for each mouse was calculated by subtraction of the weight of the left piece from that of the right piece. Comparisons of the degree of ear swelling (mg) among the groups were performed by *t*-test.

### 2.3. Test result

As shown in Table 10, the right ears of the mice in the control group significantly swell with increased thickness, and was obvious different from the left ears. The high and medium dosages of the present medication can significantly inhibit the xylene-induced ear inflammation in mice, in which the swellings of right ears in the mice reduce significantly and show significant difference as compared to the control model group (P<0.05 - P<0.01). The low dosage of the present medication has an inapparent swelling-inhibitory effect and shows no significant difference as compared to the control model group. The experimental results show that the high and medium dosages of the present medication exert better anti-inflammation effect on the xylene-induced ear swelling mouse model.

**Table 10: Effect of the present medication on xylene-induced ear swelling in mice (n =10 X̅ ±SD)**

| Group | Dosage | Animal | Swelling degree |
|---|---|---|---|
| | (/kg) | (number) | (mg) |
| Control model group | 25ml | 10 | 8.30±2.00 |
| Aspirin group | 0.2g | 10 | 3.80±0.79*** |
| JSOL group | 16g | 10 | 4.50±2.68** |
| H-dosage group | 12g | 10 | 5.40±1.84** |
| M-dosage group | 6g | 10 | 5.70±3.06* |
| L-dosage group | 3g | 10 | 7.20±2.66 |

| | | | |
|---|---|---|---|
| Note: *P<0.05; **P<0.01; ***P<0.001, as compared to the control group. | | | |

### 2.4. Summary

The experimental results show that the high and medium dosages of the present medication exert significant anti-inflammation effect on the xylene-induced ear swelling mouse model (P<0.05, P0.01).

### 3 Conclusion:

The two experimental investigations described above suggest that the present medication has an anti-inflammation effect.

### Test example 6: Investigation on the toxicology of the present medication

1. An acute toxication experiment on the present medication was performed with mice. Since the LD₅₀ could not be measured, the maximum dose was determined. The mice were intragastrically administered. The experimental result indicates that the animals have no adverse reaction and death when administered with a maximum dose of 280g crude drug/kg which is 436 times of the clinical dose (the clinical dose is 45g crude drug/person/day, assuming the weight of person being 70kg). The animals were autopsied at the end of the experiment and no pathological change in each of the organs was observed with naked eyes.
2. 120 of the wistar rats were assigned into the control group and the test groups of the present medication with dosages of 15 g, 30 g, and 60 g crude drug/kg (which are 25, 50, 100 times of the clinical dosage respectively), intragastricall administered for consecutive 12 and 24 weeks, and recovered (that is, underwent drug withdrawal for 4 weeks). Experimental results show that after administered for 12 and 24 weeks and during the recovery period, the rats in the test groups exhibit no significant differences in body weight, amount of food ingestion and clotting time as compared to those in the control group. The measured haematological indices show that the animals in the high dosage group after 12 weeks' administration and the animals in the medium dosage group during the recovery period have increased RBC and HGB values in comparison with those in the control group (P<0.05), wherein the RBC value falls within the normal rang (7.49±0.64) and the HGB value may associate with slightly higher values of a few animals. All of the other haematological indices in the test groups after 24 weeks' administration and during the recovery period show no significant difference in comparison with the control group. Reticulocyte counts in each of the test groups of 12 weeks administration, 24 weeks administration and the recovery period show no significant difference in comparison with the control group. The measured twelve blood biochemical indices show that the ALT and GLU values in the medium dosage group and the AST value in the low dosage group after 12 weeks' administration slightly increase in comparison with those in the control group (P<0.05), and all of them fall within the normal rang (being 43.15±21.84, 6.18±2.04, and 139.80±40.25, respectively, n=630). All of the other blood biochemical indices in the test groups after 24 weeks' administration and the recovery period (drug withdrawal for 4 weeks) show no significant difference in comparison with the control group. In each group of 12 weeks administration, 24 weeks administration and recovery period (drug withdrawal for 4 weeks), no abnormal electrocardiographic indices in the standard lead 11 electrocardiogram such as PR interval, QRS interval, QT interval, T wave amplitude, heart rate, etc. were observed. By gross observation in anatomy, no abnormal changes in organs of the animals from each of the groups were found and the organ indexes were of no significant difference. In the pathological examination, no apparent toxically pathological changes caused by the present medication in the organs were found.

### 3. Conclusion:

It has been demonstrated via the acute toxicity test (the maximum dose experiment) and the chronic toxicity test that the various indices in each test group show no significant difference in comparison with the control group. In the pathological examination, no apparent toxically pathological changes caused by the present medication in organs were found. Thus, the present medication is confirmed to be of no toxicity.

In conclusion, it has been confirmed from various respects such as checking cough, dispelling phlegm, relieving asthma, anti-inflammation, etc. that the present medication has good therapeutic efficacy on bronchitis, particularly has excellent therapeutic efficacy on acute tracheobronchitis.

## Claims

1. An EPHEDRAE HERBA-containing pharmaceutical composition for use in the treatment of bronchitis, **characterized in that**, said pharmaceutical composition is made from the medicinal materials in the following parts by weight:
| | |
|---|---|
| EPHEDRAE HERBA 52-86 | GYPSUM FIBROSUM 194-324 |
| FORSYTHIAE FRUCTUS 194-324 | SCUTELLARIAE RADIX 78-130 |
| MORI CORTEX 194-324 | ARMENIACAE SEMEN AMARUM 78-130 |
| PEUCEDANI RADIX 78-130 | PINELLIAE RHIZOMA78-130 |
| CITRI RETICULATAE PERICARPIUM 78-130 | FRITILLARIAE THUNBERGII BULBUS 78-130 |
| ARCTII FRUCTUS 78-130 | LONICERAE FLOS 78-130 |
| RHEI RADIX ET RHIZOMA 39-65 | PLATYCODONIS RADIX 46-76 and GLYCYRRHIZAE RADIX ET RHIZOMA 39-65. |

2. The EPHEDRAE HERBA-containing pharmaceutical composition for use according to Claim 1, **characterized in that**, said pharmaceutical composition is made from the medicinal materials in the following parts by weight:
| | |
|---|---|
| EPHEDRAE HERBA 52 | GYPSUM FIBROSUM 324 |
| FORSYTHIAE FRUCTUS 194 | SCUTELLARIAE RADIX 78 |
| MORI CORTEX 194 | ARMENIACAE SEMEN AMARUM 130 |
| PEUCEDANI RADIX 78 | PINELLIAE RHIZOMA 130 |
| CITRI RETICULATAE PERICARPIUM 78 | FRITILLARIAE THUNBERGII BULBUS 78 |
| ARCTII FRUCTUS 130 | LONICERAE FLOS 130 |
| RHEI RADIX ET RHIZOMA 39 | PLATYCODONIS RADIX 76 and GLYCYRRHIZAE RADIX ET RHIZOMA 65. |

3. The EPHEDRAE HERBA-containing pharmaceutical composition for use according to Claim 1, **characterized in that**, said pharmaceutical composition is made from the medicinal materials in the following part by weight:
| | |
|---|---|
| EPHEDRAE HERBA 86 | GYPSUM FIBROSUM 194 |
| FORSYTHIAE FRUCTUS 324 | SCUTELLARIAE RADIX130 |
| MORI CORTEX 324 | ARMENIACAE SEMEN AMARUM 78 |
| PEUCEDANI RADIX 130 | PINELLIAE RHIZOMA 78 |
| CITRI RETICULATAE PERICARPIUM 130 | FRITILLARIAE THUNBERGII BULBUS 130 |
| ARCTII FRUCTUS 78 | LONICERAE FLOS 78 |
| RHEI RADIX ET RHIZOMA 65 | PLATYCODONIS RADIX 46 and GLYCYRRHIZAE RADIX ET RHIZOMA 39. |

4. The EPHEDRAE HERBA-containing pharmaceutical composition for use according to Claim 1, **characterized in that**, said pharmaceutical composition is made from the medicinal materials in the following part by weight:
| | |
|---|---|
| EPHEDRAE HERBA 69 | GYPSUM FIBROSUM 259 |
| FORSYTHIAE FRUCTUS 259 | SCUTELLARIAE RADIX104 |
| MORI CORTEX 259 | ARMENIACAE SEMEN AMARUM 104 |
| PEUCEDANI RADIX 104 | PINELLIAE RHIZOMA 104 |
| CITRI RETICULATAE PERICARPIUM 104 | FRITILLARIAE THUNBERGII BULBUS 104 |
| ARCTII FRUCTUS 104 | LONICERAE FLOS 104 |
| RHEI RADIX ET RHIZOMA 52 | PLATYCODONIS RADIX 61 and GLYCYRRHIZAE RADIX ET RHIZOMA 52. |

5. The EPHEDRAE HERBA-containing pharmaceutical composition for use according to Claim 1, **characterized in that**, said pharmaceutical composition is made from the medicinal materials in the following part by weight:
| | |
|---|---|
| EPHEDRAE HERBA 55 | GYPSUM FIBROSUM 254 |
| FORSYTHIAE FRUCTUS 318 | SCUTELLARIAE RADIX107 |
| MORI CORTEX 203 | ARMENIACAE SEMEN AMARUM 107 |
| PEUCEDANI RADIX 82 | PINELLIAE RHIZOMA 105 |
| CITRI RETICULATAE PERICARPIUM 84 | FRITILLARIAE THUNBERGII BULBUS 125 |
| ARCTII FRUCTUS 122 | LONICERAE FLOS 113 |
| RHEI RADIX ET RHIZOMA42 | PLATYCODONIS RADIX 60 and GLYCYRRHIZAE RADIX ET RHIZOMA 50. |

6. The EPHEDRAE HERBA-containing pharmaceutical composition for use according to Claim 1, **characterized in that**, said pharmaceutical composition is made from the medicinal materials in the following part by weight:
| | |
|---|---|
| EPHEDRAE HERBA 84 | GYPSUM FIBROSUM. 313 |
| FORSYTHIAE FRUCTUS 298 | SCUTELLARIAE RADIX98 |
| MORI CORTEX 274 | ARMENIACAE SEMEN AMARUM 105 |
| PEUCEDANI RADIX 116 | PINELLIAE RHIZOMA 123 |
| CITRI RETICULATAE PERICARPIUM 95 | FRITILLARIAE THUNBERGII BULBUS 88 |
| ARCTII FRUCTUS 80 | LONICERAE FLOS 99 |
| RHEI RADIX ET RHIZOMA 63 | PLATYCODONIS RADIX 55 and GLYCYRRHIZAE RADIX ET RHIZOMA 43. |

7. The pharmaceutical composition for use according to any one of Claims 1-6, **characterized in that**, said ARMENIACAE SEMEN AMARUM is ARMENIACAE SEMEN AMARUM TOSTUM and the PINELLIAE RHIZOMA is PINELLIAE RHIZOMA PRAEPARATUM CUM ALUMINE.

8. The pharmaceutical composition for use according to any one of Claims 1-7, **characterized in that**, the pharmaceutical composition is prepared by steps of:
(1) weighing FRITILLARIAE THUNBERGII BULBUS arccording to the parts set forth in a prescription and pulverizing the same into fine powder for later use;
(2) weighing EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA according to the parts set forth in the prescription and extracting the same two times under reflux with 40-70% ethanol solution, each time for 1-4 hours, in which the amount of the ethanol solution is 8 -10 times by v/w (ml/g) of the weight of the medicinal materials for the first time and 6-9 times by v/w (ml/g) for the second time; pooling the extracts and then filtrating the mixed solution; recovering ethanol from the filtrate under a reduced pressure and concentrating at the same time to obtain a clear paste with the relative density of 1.14 - 1.16 thermally measured at 60 °C, for later use;
(3) weighing GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA according to the parts set forth in the prescription and decocting the same two times with water, each time for 1-4 hours, in which the amount of the water is 9-11 times by v/w (ml/g) of the weight of the medicinal materials for the first time and 7-9 times by v/w (ml/g) for the second time; then, pooling the decoctions, filtrating the mixed solution, concentrating the filtrate under a reduced pressure to obtain a clear paste with the relative density of 1.14-1.16 thermally measured at 60 °C, and combining the clear paste with the clear paste obtained in step (2) for later use;
the fine powder obtained in step (1) and the combined clear pastes obtained in step (3) constitutes the active component of the pharmaceutical composition.

9. The pharmaceutical composition for use according to any one of Claims 1-8, **characterized in that,** the pharmaceutical composition is in form of hard capsules, tablets, powders, oral liquids, soft capsules, pills, tinctures, syrups, suppositorys, gels, sprays or injections.

10. The pharmaceutical composition for use according to Claims 9, **characterized in that**, the tablets of the pharmaceutical composition are prepared by steps of:
(1) weighing FRITILLARIAE THUNBERGII BULBUS according to the parts set forth in a prescription and pulverizing the same into fine powder for later use;
(2) weighing EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA according to the parts set forth in the prescription and extracting the same two times under reflux with 40-70% ethanol solution, each time for 1-4 hours, in which the amount of the ethanol solution is 8-10 times by v/w (ml/g) of the weight medicinal materials for the first time and 6-9 times by v/w (ml/g) for the second time; pooling the extracts and filtrating the mixed solution; recovering ethanol from the filtrate under a reduced pressure and concentrating at the same time to obtain a clear paste with the relative density of 1.14-1.16 thermally measured at 60 °C, for later use;
(3) weighing GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA according to the parts set forth in the prescription and decocting two times with water, each time for 1-4 hours, in which the amount of the water is 9-11 times by v/w (ml/g) of the weight of the medicinal materials for the first time and 7-9 times by v/w (ml/g) for the second time; then, pooling the decoctions and filtrating the mixed solution, concentrating the filtrate under a reduced pressure to obtain a clear paste with the relative density of 1.14-1.16 thermally measured at 60 °C, and combined the clear paste with the clear paste obtained in step (2) for later use;
(4) spray drying the combined clear pastes obtained in step (3) and collecting the atomized powder for later use;
(5) providing materials in parts by weight for tableting as follows:
| | |
|---|---|
| atomized powder obtained in step (4) 200-355; | fine powder obtained in step (1) 54-145; |
| carboxymethylstarch sodium 10-15.5; | microcrystalline cellulose 7-12; |
| magnesium stearate 1.5-3.5; | starch proper amount; |
(6) tableting according to conventional formulation process.

11. The pharmaceutical composition for use according to Claims 10, **characterized in that**, the tablets of the pharmaceutical composition are prepared by steps of:
(1) weighing FRITILLARIAE THUNBERGII BULBUS according to the parts set forth in a prescription and pulverizing the same into fine powder for later use;
(2) weighing EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, and RHEI RADIX ET RHIZOMA according to the parts set forth in the prescription and extracting two times under reflux with 50% ethanol solution, each time for 3 hours, in which the amount of the ethanol solution is 10 times by v/w (ml/g) of the weight of the medicinal materials for the first time and 6 times by v/w (ml/g) for the second time; pooling the extracts and filtrating the mixed solution; recovering ethanol from the filtrate under a reduced pressure and concentrating at the same time to obtain a clear paste with the relative density of 1.15 thermally measured at 60 °C, for later use;
(3) weighing GYPSUM FIBROSUM, MORI CORTEX, PEUCEDANI RADIX, CITRI RETICULATAE PERICARPIUM, LONICERAE FLOS, PLATYCODONIS RADIX, and GLYCYRRHIZAE RADIX ET RHIZOMA according to the parts set forth in the prescription and decocting two times with water, each time for 2 hours, in which the amount of the added water is 10 times by v/w (ml/g) of the weight of medicinal materials for the first time and 7 times by v/w (ml/g) for the second time; then, pooling the decoctions, filtrating the mixed solution, concentrating the filtrate under a reduced pressure to obtain a clear paste with the relative density of 1.15 thermally measured at 60 °C, and combined the clear paste with the clear paste obtained in step (2) for later use;
(4) spray drying the combined clear pastes obtained in step (3) and collecting the atomized powder for later use;
(5) providing materials in parts by weight for tableting as follows:
atomized powder obtained in step (4) 282.6; fine powder obtained in step (1) 101;
carboxymethylstarch sodium 12.5; microcrystalline cellulose 9.0;
magnesium stearate 2.25; starch proper amount;
(6) tableting according to conventional formulation process.

12. The EPHEDRAE HERBA-containing pharmaceutical composition for use in the treament of bronchitis according to any one of Claims 1-11, **characterized in that**, said bronchitis is an acute tracheobronchitis.

## Patentansprüche

1. EPHEDRAE HERBA-haltige pharmazeutische Zusammensetzung zur Verwendung bei der Behandlung von Bronchitis, **dadurch gekennzeichnet, dass** die pharmazeutische Zusammensetzung aus den medizinischen Materialien mit den folgenden Gewichtsanteilen hergestellt ist:
| | |
|---|---|
| EPHEDRAE HERBA | GYPSUM FIBROSUM |
| 52 - 86 | 194 - 324 |
| | |
| FORSYTHIAE FRUCTUS | SCUTELLARIAE RADIX |
| 194 - 324 | 78 - 130 |
| | |
| MORI CORTEX | ARMENIACAE SEMEN AMARUM |
| 194 - 324 | 78 - 130 |
| | |
| PEUCEDANI RADIX | PINELLIAE RHIZOMA |
| 78 - 130 | 78 - 130 |
| | |
| CITRI RETICULATAE PERICARPIUM | FRITILLARIAE THUNBERGII BULBUS |
| 78 - 130 | 78 - 130 |
| | |
| ARCTII FRUCTUS | LONICERAE FLOS |
| 78 - 130 | 78 - 130 |
| | |
| RHEI RADIX ET RHIZOMA | PLATYCODONIS RADIX |
| 39 - 65 | 46 - 76 und |
| | |
| GLYCYRRHIZAE RADIX ET RHIZOMA | |
| 39-65. | |

2. EPHEDRAE HERBA-haltige pharmazeutische Zusammensetzung zur Verwendung nach Anspruch 1, **dadurch gekennzeichnet, dass** die pharmazeutische Zusammensetzung aus den medizinischen Materialien mit den folgenden Gewichtsanteilen hergestellt ist:
| | |
|---|---|
| EPHEDRAE HERBA | GYPSUM FIBROSUM |
| 52 | 324 |
| | |
| FORSYTHIAE FRUCTUS | SCUTELLARIAE RADIX |
| 194 | 78 |
| | |
| MORI CORTEX | ARMENIACAE SEMEN AMARUM |
| 194 | 130 |
| | |
| PEUCEDANI RADIX | PINELLIAE RHIZOMA |
| 78 | 130 |
| | |
| CITRI RETICULATAE PERICARPIUM | FRITILLARIAE THUNBERGII BULBUS |
| 78 | 78 |
| ARCTII FRUCTUS | LONICERAE FLOS |
| 130 | 130 |
| | |
| RHEI RADIX ET RHIZOMA | PLATYCODONIS RADIX |
| 39 | 76 und |
| | |
| GLYCYRRHIZAE RADIX ET RHIZOMA | |
| 65. | |

3. EPHEDRAE HERBA-haltige pharmazeutische Zusammensetzung zur Verwendung nach Anspruch 1, **dadurch gekennzeichnet, dass** die pharmazeutische Zusammensetzung aus den medizinischen Materialien mit den folgenden Gewichtsanteilen hergestellt ist:
| | |
|---|---|
| EPHEDRAE HERBA | GYPSUM FIBROSUM |
| 86 | 194 |
| | |
| FORSYTHIAE FRUCTUS | SCUTELLARIAE RADIX |
| 324 | 130 |
| | |
| MORI CORTEX | ARMENIACAE SEMEN AMARUM |
| 324 | 78 |
| | |
| PEUCEDANI RADIX | PINELLIAE RHIZOMA |
| 130 | 78 |
| | |
| CITRI RETICULATAE PERICARPIUM | FRITILLARIAE THUNBERGII BULBUS |
| 130 | 130 |
| | |
| ARCTII FRUCTUS | LONICERAE FLOS |
| 78 | 78 |
| | |
| RHEI RADIX ET RHIZOMA | PLATYCODONIS RADIX |
| 65 | 46 und |
| | |
| GLYCYRRHIZAE RADIX ET RHIZOMA | |
| 39. | |

4. EPHEDRAE HERBA-haltige pharmazeutische Zusammensetzung zur Verwendung nach Anspruch 1, **dadurch gekennzeichnet, dass** die pharmazeutische Zusammensetzung aus den medizinischen Materialien mit den folgenden Gewichtsanteilen hergestellt ist:
| | |
|---|---|
| EPHEDRAE HERBA | GYPSUM FIBROSUM |
| 69 | 259 |
| | |
| FORSYTHIAE FRUCTUS | SCUTELLARIAE RADIX |
| 259 | 104 |
| MORI CORTEX | ARMENIACAE SEMEN AMARUM |
| 259 | 104 |
| | |
| PEUCEDANI RADIX | PINELLIAE RHIZOMA |
| 104 | 104 |
| | |
| CITRI RETICULATAE PERICARPIUM | FRITILLARIAE THUNBERGII BULBUS |
| 104 | 104 |
| | |
| ARCTII FRUCTUS | LONICERAE FLOS |
| 104 | 104 |
| | |
| RHEI RADIX ET RHIZOMA | PLATYCODONIS RADIX |
| 52 | 61 und |
| | |
| GLYCYRRHIZAE RADIX ET RHIZOMA | |
| 52. | |

5. EPHEDRAE HERBA-haltige pharmazeutische Zusammensetzung zur Verwendung nach Anspruch 1, **dadurch gekennzeichnet, dass** die pharmazeutische Zusammensetzung aus den medizinischen Materialien mit den folgenden Gewichtsanteilen hergestellt ist:
| | |
|---|---|
| EPHEDRAE HERBA | GYPSUM FIBROSUM |
| 55 | 254 |
| | |
| FORSYTHIAE FRUCTUS | SCUTELLARIAE RADIX |
| 318 | 107 |
| | |
| MORI CORTEX | ARMENIACAE SEMEN AMARUM |
| 203 | 107 |
| | |
| PEUCEDANI RADIX | PINELLIAE RHIZOMA |
| 82 | 105 |
| CITRI RETICULATAE PERICARPIUM | FRITILLARIAE THUNBERGII BULBUS |
| 84 | 125 |
| | |
| ARCTII FRUCTUS | LONICERAE FLOS |
| 122 | 113 |
| | |
| RHEI RADIX ET RHIZOMA | PLATYCODONIS RADIX |
| 42 | 60 und |
| | |
| GLYCYRRHIZAE RADIX ET RHIZOMA | |
| 50. | |

6. EPHEDRAE HERBA-haltige pharmazeutische Zusammensetzung zur Verwendung nach Anspruch 1, **dadurch gekennzeichnet, dass** die pharmazeutische Zusammensetzung aus den medizinischen Materialien mit den folgenden Gewichtsanteilen hergestellt ist:
| | |
|---|---|
| EPHEDRAE HERBA | GYPSUM FIBROSUM |
| 84 | 313 |
| | |
| FORSYTHIAE FRUCTUS | SCUTELLARIAE RADIX |
| 298 | 98 |
| | |
| MORI CORTEX | ARMENIACAE SEMEN AMARUM |
| 274 | 105 |
| | |
| PEUCEDANI RADIX | PINELLIAE RHIZOMA |
| 116 | 123 |
| | |
| CITRI RETICULATAE PERICARPIUM | FRITILLARIAE THUNBERGII BULBUS |
| 95 | 88 |
| | |
| ARCTII FRUCTUS | LONICERAE FLOS |
| 80 | 99 |
| RHEI RADIX ET RHIZOMA | PLATYCODONIS RADIX |
| 63 | 55 und |
| | |
| GLYCYRRHIZAE RADIX ET RHIZOMA | |
| 43. | |

7. Pharmazeutische Zusammensetzung zur Verwendung nach einem der Ansprüche 1 - 6, **dadurch gekennzeichnet, dass**: ARMENIACAE SEMEN AMARUM ist ARMENIACAE SEMEN AMARUM TOSTUM und das PINELLIAE RHIZOMA ist PINELLIAE RHIZOMA PRAEPARATUM CUM ALUMINE.

8. Pharmazeutische Zusammensetzung zur Verwendung nach einem der Ansprüche 1 - 7, **dadurch gekennzeichnet, dass** die pharmazeutische Zusammensetzung durch die Schritte hergestellt wird:
(1) Wiegen von FRITILLARIAE THUNBERGII BULBUS entsprechend den in der Beschreibung dargelegten Teilen und Pulverisieren desselben zu einem feinen Pulver für die spätere Verwendung;
(2) Wiegen von EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS und RHEI RADIX ET RHIZOMA entsprechend den in der Beschreibung dargelegten Teilen und zweimaliges Extrahieren derselben unter Reflux mit einer 40 - 70 % Ethanollösung, jedes Mal für 1 - 4 Stunden, wobei beim ersten Mal die Menge der Ethanollösung 8 - 10-mal v/w (ml/g) das Gewicht von den medizinischen Materialien ist und beim zweiten Mal 6 - 9-mal v/w (ml/g); Vereinen der Extrakte und dann Filtrieren der gemischten Lösung; Rückgewinnen des Ethanols aus dem Filtrat unter einem reduziertem Druck und gleichzeitiges Konzentrieren, um eine klare Paste mit der relativen Dichte von 1,14 - 1,16, thermisch bei 60 °C gemessen, für die spätere Verwendung zu erhalten;
(3) Wiegen von GYPSUM FIBROSUM, MORI CORTEX, PEUCEDANI RADIX, CITRI RETICULATAE PERICARPIUM, LONICERAE FLOS, PLATYCODONIS RADIX und GLYCYRRHIZAE RADIX ET RHIZOMA entsprechend den in der Beschreibung dargelegten Teilen und zweimaliges Absieden derselben mit Wasser, jedes Mal für 1 - 4 Stunden, wobei beim ersten Mal die Wassermenge 9 - 11-mal v/w (ml/g) das Gewicht von den medizinischen Materialien ist und beim zweiten Mal 7 - 9-mal v/w (ml/g); dann Vereinen der Absude, Filtrieren der gemischten Lösung, Konzentrieren des Filtrats unter einem reduziertem Druck, um eine klare Paste mit der relativen Dichte von 1,14 - 1,16, thermisch bei 60 °C gemessen, zu erhalten und Kombinieren der klaren Paste mit der klaren Paste, die im Schritt (2) erhalten wird, für die spätere Verwendung; wobei das feine Pulver, das im Schritt (1) erhalten wird, und die kombinierten klaren Plasten, die im Schritt (3) erhalten werden, die aktiven Bestandteile der pharmazeutischen Zusammensetzung bilden.

9. Pharmazeutische Zusammensetzung zur Verwendung nach einem der Ansprüche 1 - 8, **dadurch gekennzeichnet, dass** die pharmazeutische Zusammensetzung in einer Dosierungsform von Hartkapseln, Tabletten, Pulvern, Oralflüssigkeiten, Weichkapseln, Pillen, Tinkturen, Sirups, Suppositorien, Gels, Sprays oder Injektionen ist.

10. Pharmazeutische Zusammensetzung zur Verwendung nach Anspruch 9, **dadurch gekennzeichnet, dass** die Tabletten der pharmazeutischen Zusammensetzung durch die Schritte hergestellt werden:
(1) Wiegen von FRITILLARIAE THUNBERGII BULBUS entsprechend den in der Beschreibung dargelegten Teilen und Pulverisieren desselben zu einem feinen Pulver für die spätere Verwendung;
(2) Wiegen von EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS und RHEI RADIX ET RHIZOMA entsprechend den in der Beschreibung dargelegten Teilen und zweimaliges Extrahieren derselben unter Reflux mit einer 40-70 % Ethanollösung, jedes Mal für 1 - 4 Stunden, wobei beim ersten Mal die Menge der Ethanollösung 8-10-mal v/w (ml/g) das Gewicht von den medizinischen Materialien ist und beim zweiten Mal 6 - 9-mal v/w (ml/g); Vereinen der Extrakte und Filtrieren der gemischten Lösung; Rückgewinnen des Ethanols aus dem Filtrat unter einem reduziertem Druck und gleichzeitiges Konzentrieren, um eine klare Paste mit der relativen Dichte von 1,14 - 1,16, thermisch bei 60 °C gemessen, für die spätere Verwendung zu erhalten;
(3) Wiegen von GYPSUM FIBROSUM, MORI CORTEX, PEUCEDANI RADIX, CITRI RETICULATAE PERICARPIUM, LONICERAE FLOS, PLATYCODONIS RADIX und GLYCYRRHIZAE RADIX ET RHIZOMA entsprechend den in der Beschreibung dargelegten Teilen und zweimaliges Absieden derselben mit Wasser, jedes Mal für 1 - 4 Stunden, wobei beim ersten Mal Wassermenge 9 - 11-mal v/w (ml/g) das Gewicht von den medizinischen Materialien ist und beim zweiten Mal 7 - 9-mal v/w (ml/g); dann Vereinen der Absude und Filtrieren der gemischten Lösung, Konzentrieren des Filtrats unter einem reduziertem Druck, um eine klare Paste mit der relativen Dichte von 1,14 - 1,16, thermisch bei 60 °C gemessen, zu erhalten und Kombinieren der klaren Paste mit der klaren Paste, die im Schritt (2) erhalten wird, für die spätere Verwendung;
(4) Sprühtrocknen der kombinierten klaren Pasten, die im Schritt (3) erhalten werden, und Sammeln des atomisierten Pulvers für die spätere Verwendung;
(5) Bereitstellen von Materialien in Gewichtsprozent für die Tablettierung und zwar wie folgt:
| | |
|---|---|
| atomisiertes Pulver, erhalten im Schritt (4) | 200 - 355; |
| feines Pulver, erhalten im Schritt (1) | 54 - 145; |
| Natriumcarboxymethylstärke | 10 - 15,5; |
| mikrokristalline Cellulose | 7 - 12; |
| Magnesiumstearat | 1,5 - 3,5; |
| Stärke | geeignete Menge; |
(6) Tablettieren gemäß herkömmlichen Formulierungsverfahrens.

11. Pharmazeutische Zusammensetzung zur Verwendung nach Anspruch 10, **dadurch gekennzeichnet, dass** die Tabletten der pharmazeutischen Zusammensetzung durch die Schritte hergestellt werden:
(1) Wiegen von FRITILLARIAE THUNBERGII BULBUS entsprechend den in der Beschreibung dargelegten Teilen und Pulverisieren desselben zu einem feinen Pulver für die spätere Verwendung;
(2) Wiegen von EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS und RHEI RADIX ET RHIZOMA entsprechend den in der Beschreibung dargelegten Teilen und zweimaliges Extrahieren derselben unter Reflux mit einer 50 % Ethanollösung, jedes Mal für 3 Stunden, wobei beim ersten Mal die Menge der Ethanollösung 10-mal v/w (ml/g) das Gewicht von den medizinischen Materialien ist und beim zweiten Mal 6-mal v/w (ml/g); Vereinen der Extrakte und Filtrieren der gemischten Lösung; Rückgewinnen des Ethanols aus dem Filtrat unter einem reduziertem Druck und gleichzeitiges Konzentrieren, um eine klare Paste mit der relativen Dichte von 1,15, thermisch bei 60 °C gemessen, für die spätere Verwendung zu erhalten;
(3) Wiegen von GYPSUM FIBROSUM, MORI CORTEX, PEUCEDANI RADIX, CITRI RETICULATAE PERICARPIUM, LONICERAE FLOS, PLATYCODONIS RADIX und GLYCYRRHIZAE RADIX ET RHIZOMA entsprechend den in der Beschreibung dargelegten Teilen und zweimaliges Absieden derselben mit Wasser, jedes Mal für 2 Stunden, wobei beim ersten Mal die Wassermenge 10-mal v/w (ml/g) das Gewicht von den medizinischen Materialien ist und beim zweiten Mal 7-mal v/w (ml/g); dann Vereinen der Absude, Filtrieren der gemischten Lösung, Konzentrieren des Filtrats unter einem reduziertem Druck, um eine klare Paste mit der relativen Dichte von 1,15, thermisch bei 60 °C gemessen, zu erhalten und Kombinieren der klaren Paste mit der klaren Paste, die im Schritt (2) erhalten wird, für die spätere Verwendung;
(4) Sprühtrocknen der kombinierten klaren Pasten, die im Schritt (3) erhalten werden, und Sammeln des atomisierten Pulvers für die spätere Verwendung;
(5) Bereitstellen von Materialien in Gewichtsprozent für die Tablettierung und zwar wie folgt:
| | |
|---|---|
| atomisiertes Pulver, erhalten im Schritt (4) | 282,6; |
| feines Pulver, erhalten im Schritt (1) | 101; |
| Natriumcarboxymethylstärke | 12,5; |
| mikrokristalline Cellulose | 9,0; |
| Magnesiumstearat | 2,25; |
| Stärke | geeignete Menge; |
(6) Tablettieren gemäß herkömmlichen Formulierungsverfahren.

12. EPHEDRAE HERBA-haltige pharmazeutische Zusammensetzung zur Verwendung bei der Behandlung von Bronchitis nach einem der Ansprüche 1 - 11, **dadurch gekennzeichnet, dass** die Bronchitis eine akute Tracheobronchitis ist.

## Revendications

1. Composition pharmaceutique contenant EPHEDRAE HERBA pour utilisation dans le traitement de la bronchite, **caractérisée en ce que**, ladite composition pharmaceutique est constituée des matériaux médicinaux dans les parties en poids suivantes :
| | |
|---|---|
| EPHEDRAE HERBA 52-86 | GYPSUM FIBROSUM 194-324 |
| FORSYTHIAE FRUCTUS 194-324 | SCUTELLARIAE RADIX 78-130 |
| MORI CORTEX 194-324 | ARMENIACAE SEMEN AMARUM 78-130 |
| PEUCEDANI RADIX 78-130 | PINELLIAE RHIZOMA 78-130 |
| CITRI RETICULATAE PERICARPIUM 78-130 | FRITILLARIAE THUNBERGII BULBUS 78-130 |
| ARCTII FRUCTUS 78-130 | LONICERAE FLOS 78-130 |
| RHEI RADIX ET RHIZOMA 39-65 | PLATYCODONIS RADIX 46-76 et |
| GLYCYRRHIZAE RADIX ET RHIZOMA 39-65. | |

2. Composition pharmaceutique contenant EPHEDRAE HERBA pour utilisation selon la revendication 1, **caractérisée en ce que**, ladite composition pharmaceutique est constituée des matériaux médicinaux dans les parties en poids suivantes :
| | |
|---|---|
| EPHEDRAE HERBA 52 | GYPSUM FIBROSUM 324 |
| FORSYTHIAE FRUCTUS 194 | SCUTELLARIAE RADIX 78 |
| MORI CORTEX 194 | ARMENIACAE SEMEN AMARUM 130 |
| PEUCEDANI RADIX 78 | PINELLIAE RHIZOMA 130 |
| CITRI RETICULATAE PERICARPIUM 78 | FRITILLARIAE THUNBERGII BULBUS 78 |
| ARCTII FRUCTUS 130 | LONICERAE FLOS 130 |
| RHEI RADIX ET RHIZOMA 39 | PLATYCODONIS RADIX 76 et |
| GLYCYRRHIZAE RADIX ET RHIZOMA 65. | |

3. Composition pharmaceutique contenant EPHEDRAE HERBA pour utilisation selon la revendication 1, **caractérisée en ce que**, ladite composition pharmaceutique est constituée des matériaux médicinaux dans les parties en poids suivantes :
| | |
|---|---|
| EPHEDRAE HERBA 86 | GYPSUM FIBROSUM 194 |
| FORSYTHIAE FRUCTUS 324 | SCUTELLARIAE RADIX130 |
| MORI CORTEX 324 | ARMENIACAE SEMEN AMARUM 78 |
| PEUCEDANI RADIX 130 | PINELLIAE RHIZOMA 78 |
| CITRI RETICULATAE PERICARPIUM 130 | FRITILLARIAE THUNBERGII BULBUS 130 |
| ARCTII FRUCTUS 78 | LONICERAE FLOS 78 |
| RHEI RADIX ET RHIZOMA 65 | PLATYCODONIS RADIX 46 et |

4. Composition pharmaceutique contenant EPHEDRAE HERBA pour utilisation selon la revendication 1, **caractérisée en ce que**, ladite composition pharmaceutique est constituée des matériaux médicinaux dans les parties en poids suivantes :
| | |
|---|---|
| EPHEDRAE HERBA 69 | GYPSUM FIBROSUM 259 |
| FORSYTHIAE FRUCTUS 259 | SCUTELLARIAE RADIX 104 |
| MORI CORTEX 259 | ARMENIACAE SEMEN AMARUM 104 |
| PEUCEDANI RADIX 104 | PINELLIAE RHIZOMA 104 |
| CITRI RETICULATAE PERICARPIUM 104 | FRITILLARIAE THUNBERGII BULBUS 104 |
| ARCTII FRUCTUS 104 | LONICERAE FLOS 104 |
| RHEI RADIX ET RHIZOMA 52 | PLATYCODONIS RADIX 61 et |
| GLYCYRRHIZAE RADIX ET RHIZOMA 52. | |

5. Composition pharmaceutique contenant EPHEDRAE HERBA pour utilisation selon la revendication 1, **caractérisée en ce que**, ladite composition pharmaceutique est constituée des matériaux médicinaux dans les parties en poids suivantes :
| | |
|---|---|
| EPHEDRAE HERBA 55 | GYPSUM FIBROSUM 254 |
| FORSYTHIAE FRUCTUS 318 | SCUTELLARIAE RADIX 107 |
| MORI CORTEX 203 | ARMENIACAE SEMEN AMARUM 107 |
| PEUCEDANI RADIX 82 | PINELLIAE RHIZOMA 105 |
| CITRI RETICULATAE PERICARPIUM 84 | FRITILLARIAE THUNBERGII BULBUS 125 |
| ARCTII FRUCTUS 122 | LONICERAE FLOS 113 |
| RHEI RADIX ET RHIZOMA 42 | PLATYCODONIS RADIX 60 et |
| GLYCYRRHIZAE RADIX ET RHIZOMA 50. | |

6. Composition pharmaceutique contenant EPHEDRAE HERBA pour utilisation selon la revendication 1, **caractérisée en ce que**, ladite composition pharmaceutique est constituée des matériaux médicinaux dans les parties en poids suivantes :
| | |
|---|---|
| EPHEDRAE HERBA 84 | GYPSUM FIBROSUM 313 |
| FORSYTHIAE FRUCTUS 298 | SCUTELLARIAE RADIX 98 |
| MORI CORTEX 274 | ARMENIACAE SEMEN AMARUM 105 |
| PEUCEDANI RADIX 116 | PINELLIAE RHIZOMA 123 |
| CITRI RETICULATAE PERICARPIUM 95 | FRITILLARIAE THUNBERGII BULBUS 88 |
| ARCTII FRUCTUS 80 | LONICERAE FLOS 99 |
| RHEI RADIX ET RHIZOMA 63 | PLATYCODONIS RADIX 55 et |
| GLYCYRRHIZAE RADIX ET RHIZOMA 43. | |

7. Composition pharmaceutique pour utilisation selon l'une quelconque des revendications 1 à 6, **caractérisée en ce que**, ledit ARMENIACAE SEMEN AMARUM est ARMENIACAE SEMEN AMARUM TOSTUM et le PINELLIAE RHIZOMA est PINELLIAE RHIZOMA PRAEPARATUM CUM ALUMINE.

8. Composition pharmaceutique pour utilisation selon l'une quelconque des revendications 1 à 7, **caractérisée en ce que**, la composition pharmaceutique est préparée par les étapes de :
(1) pesée de FRITILLARIAE THUNBERGII BULBUS conformément aux parties décrites dans une prescription et pulvérisation de celui-ci en poudre fine pour utilisation ultérieure ;
(2) pesée d'EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, et RHEI RADIX ET RHIZOMA conformément aux parties décrites dans la prescription et extraction de ceux-ci deux fois à reflux avec une solution d'éthanol à 40-70 %, chaque fois pendant 1 à 4 heures, où la quantité de la solution d'éthanol est 8 à 10 fois en v/m (ml/g) du poids de matériaux médicinaux pour la première fois et 6 à 9 fois en v/m (ml/g) pour la seconde fois ; groupement des extraits et ensuite filtration de la solution mélangée ; récupération de l'éthanol à partir du filtrat sous pression réduite et concentration simultanément pour obtenir une pâte limpide ayant une densité de 1,14 à 1,16 mesurée thermiquement à 60 °C, pour utilisation ultérieure ;
(3) pesée de GYPSUM FIBROSUM, MORI CORTEX, PEUCEDANI RADIX, CITRI RETICULATAE PERICARPIUM, LONICERAE FLOS, PLATYCODONIS RADIX, et GLYCYRRHIZAE RADIX ET RHIZOMA conformément aux parties décrites dans la prescription et décoction les mêmes deux fois avec de l'eau, chaque fois pendant 1 à 4 heures, où la quantité de l'eau est de 9 à 11 fois en v/m (ml/g) du poids des matériaux médicinaux pour la première fois et 7 à 9 fois en v/m (ml/g) pour la seconde fois ; puis, groupement des décoctions, filtration de la solution mélangée, concentration du filtrat sous pression réduite pour obtenir une pâte limpide ayant une densité de 1,14 à 1,16 mesurée thermiquement à 60 °C, et combinaison de la pâte limpide avec la pâte limpide obtenue dans l'étape (2) pour utilisation ultérieure,
la poudre fine obtenue dans l'étape (1) et les pâtes limpides combinées obtenues dans l'étape (3) constituent le composant actif de la composition pharmaceutique.

9. Composition pharmaceutique pour utilisation selon l'une quelconque des revendications 1 à 8, **caractérisée en ce que**, la composition pharmaceutique est sous la forme de gélules, comprimés, poudres, liquides oraux, capsules molles, pilules, teintures, sirops, suppositoires, gels, aérosols ou injections.

10. Composition pharmaceutique pour utilisation selon la revendication 9, **caractérisée en ce que**, la composition pharmaceutique est préparée par les étapes de:
(1) pesée de FRITILLARIAE THUNBERGII BULBUS conformément aux parties décrites dans une prescription et pulvérisation de celui-ci en poudre fine pour utilisation ultérieure ;
(2) pesée d'EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, et RHEI RADIX ET RHIZOMA conformément aux parties décrites dans la prescription et extraction de ceux-ci deux fois à reflux avec une solution d'éthanol à 40-70 %, chaque fois pendant 1 à 4 heures, où la quantité de la solution d'éthanol est 8 à 10 fois en v/m (ml/g) du poids de matériaux médicinaux pour la première fois et 6 à 9 fois en v/m (ml/g) pour la seconde fois ; groupement des extraits et filtration de la solution mélangée ; récupération de l'éthanol à partir du filtrat sous pression réduite et concentration simultanément pour obtenir une pâte limpide ayant une densité de 1,14 à 1,16 mesurée thermiquement à 60 °C, pour utilisation ultérieure ;
(3) pesée de GYPSUM FIBROSUM, MORI CORTEX, PEUCEDANI RADIX, CITRI RETICULATAE PERICARPIUM, LONICERAE FLOS, PLATYCODONIS RADIX, et GLYCYRRHIZAE RADIX ET RHIZOMA conformément aux parties décrites dans la prescription et décoction deux fois avec de l'eau, chaque fois pendant 1 à 4 heures, où la quantité de l'eau est de 9 à 11 fois en v/m (ml/g) du poids des matériaux médicinaux pour la première fois et 7 à 9 fois en v/m (ml/g) pour la seconde fois ; puis, groupement des décoctions et filtration de la solution mélangée, concentration du filtrat sous pression réduite pour obtenir une pâte limpide ayant une densité de 1,14 à 1,16 mesurée thermiquement à 60 °C, et combinaison de la pâte limpide avec la pâte limpide obtenue dans l'étape (2) pour utilisation ultérieure ;
(4) séchage par pulvérisation des pâtes limpides combinées obtenues dans l'étape (3) et collecte de la poudre atomisée pour utilisation ultérieure ;
(5) fourniture des matériaux en partie en poids pour fabrication de comprimés comme suit :
| | |
|---|---|
| poudre atomisée obtenue dans l'étape (4) 200-355 ; | poudre fine obtenue dans l'étape (1) 54-145 ; |
| carboxyméthylamidon sodique 10-15,5 ; | cellulose microcristalline 7-12 ; |
| stéarate de magnésium 1,5-3,5 ; | quantité appropriée d'amidon ; |
(6) fabrication de comprimé selon un procédé de formulation conventionnel.

11. Composition pharmaceutique pour utilisation selon la revendication 10, **caractérisée en ce que**, les comprimés de la composition pharmaceutique sont préparés par les étapes de :
(1) pesée de FRITILLARIAE THUNBERGII BULBUS conformément aux parties décrites dans une prescription et pulvérisation de celui-ci en poudre fine pour utilisation ultérieure ;
(2) pesée d'EPHEDRAE HERBA, FORSYTHIAE FRUCTUS, SCUTELLARIAE RADIX, ARMENIACAE SEMEN AMARUM, PINELLIAE RHIZOMA, ARCTII FRUCTUS, et RHEI RADIX ET RHIZOMA conformément aux parties décrites dans la prescription et extraction de ceux-ci deux fois à reflux avec une solution d'éthanol à 50 %, chaque fois pendant 3 heures, où la quantité de la solution d'éthanol est 10 fois en v/m (ml/g) du poids des matériaux médicinaux pour la première fois et 6 fois en v/m (ml/g) pour la seconde fois ; groupement des extraits et filtration de la solution mélangée ; récupération de l'éthanol à partir du filtrat sous pression réduite et concentration simultanément pour obtenir une pâte limpide ayant une densité de 1,15 mesurée thermiquement à 60 °C, pour utilisation ultérieure ;
(3) pesée de GYPSUM FIBROSUM, MORI CORTEX, PEUCEDANI RADIX, CITRI RETICULATAE PERICARPIUM, LONICERAE FLOS, PLATYCODONIS RADIX, et GLYCYRRHIZAE RADIX ET RHIZOMA conformément aux parties décrites dans la prescription et décoction deux fois avec de l'eau, chaque fois pendant 2 heures, où la quantité de l'eau ajoutée est 10 fois en v/m (ml/g) du poids des matériaux médicinaux pour la première fois et 7 fois en v/m (ml/g) pour la seconde fois ; puis, groupement des décoctions, filtration de la solution mélangée, concentration du filtrat sous pression réduite pour obtenir une pâte limpide ayant une densité de 1,15 mesurée thermiquement à 60 °C, et combinaison de la pâte limpide avec la pâte limpide obtenue dans l'étape (2) pour utilisation ultérieure ;
(4) séchage par pulvérisation des pâtes limpides combinées obtenues dans l'étape (3) et collecte de la poudre atomisée pour utilisation ultérieure ;
(5) fourniture des matériaux en partie en poids pour fabrication de comprimés comme suit :
| | |
|---|---|
| poudre atomisée obtenue dans l'étape (4) 282,6 ; | poudre fine obtenue dans l'étape (1) 101 ; |
| carboxyméthylamidon sodique 12,5 ; | cellulose microcristalline 9,0 ; |
| stéarate de magnésium 2,25 ; | quantité appropriée d'amidon ; |
(6) fabrication de comprimé selon un procédé de formulation conventionnel.

12. Composition pharmaceutique contenant EPHEDRAE HERBA pour utilisation dans le traitement de la bronchite selon l'une quelconque des revendications 1 à 11, **caractérisée en ce que**, ladite bronchite est une trachéo-bronchite aiguë.
